(19) Europäisches Patentamt / European Patent Office / Office européen des brevets

(11) **EP 4 162 879 A1**

(12) **EUROPEAN PATENT APPLICATION**

(43) Date of publication:
**12.04.2023 Bulletin 2023/15**

(21) Application number: **21290060.9**

(22) Date of filing: **05.10.2021**

(51) International Patent Classification (IPC):
**A61B 6/00** (2006.01)

(52) Cooperative Patent Classification (CPC):
**A61B 6/504; A61B 6/481; A61B 6/486;
A61B 6/507; A61B 6/5217**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
- **Florent, Raoul
  5656 AE Eindhoven (NL)**
- **Levrier, Claire
  5656 AE Eindhoven (NL)**
- **This, Alexandre
  5656 AE Eindhoven (NL)**
- **Schmitt, Holger
  5656 AE Eindhoven (NL)**
- **Van der Horst, Arjen
  5656 AE Eindhoven (NL)**

(54) **DETERMINING VESSEL PARAMETERS**

(57) A system (100) for providing value of a transit velocity of an injected contrast agent bolus in a lumen, is provided. The system comprises one or more processors (120) configured to: receive X-ray angiographic image data comprising a temporal sequence of images representing a flow of the injected contrast agent bolus through a vessel tree comprising the lumen; generate a time-intensity curve for the vessel tree from the temporal sequence of images; calculate a transit time ($T_T$) from the time-intensity curve; determine an estimate of the transit length ($d_T$) of the lumen based on an analysis of an earlier X-ray image and a later X-ray image; and calculate the value of the transit velocity of the injected contrast agent bolus in the lumen based on a ratio of the estimated transit length ($d_T$) to the calculated transit time ($T_T$).

FIG. 2

EP 4 162 879 A1

**Description**

Technical Field

The present disclosure relates to the medical field, and more particularly to the determination of vessel parameters. A first aspect of the present disclosure relates to computing a microcirculatory resistance value for a vessel. A second aspect of the present disclosure relates to providing a transit time for a vessel. A third aspect of the present disclosure relates to computing a length of a portion of a vessel from X-ray projection image data. A system, a computer-implemented method, and a computer program product, are disclosed for each of these aspects.

Background

Coronary Artery Diseases "CAD" refers to pathologies of the coronary arteries and to the deterioration of their ability to transport oxygenated blood to the heart muscle. Ultimately, the lack of oxygen supply results in myocardial ischemia, whose symptoms can include shortness of breath, angina, or even myocardial infarction.

While obstructive CAD is well recognized as a contributing factor to the reduction of blood supply to the heart muscle, there is an increasing number of patients with evidence of ischemia and/or reports of chest pain without obstructive CAD, also referred to as Non-Obstructive Coronary Artery Disease "NOCAD".

Coronary Microvascular Dysfunction "CMVD", or in short, MVD, has been shown to be a contributing factor to myocardial ischemia and myocardial dysfunction, especially in the NOCAD context. As such, there is a great interest in assessing coronary microvasculature.

Several flow parameters have been proposed for use in evaluating the microvasculature. These include the Coronary Flow Reserve "CFR", the Thrombolysis in Myocardial Infarction "TIMI" flow grade, TIMI frame count, and the Index of Microcirculatory Resistance "IMR". The Index of Microcirculatory Resistance is also known under the same abbreviation as the Index of Microvascular Resistance. The IMR is usually recognized as being more specific and informative as compared to CFR or TIMI methods. The IMR is usually measured invasively using a pressure/ flow-wire, although more recently, angiography-derived measurements have been investigated.

The computation of microcirculatory resistance values, such as the IMR, is based on the measurement of a transit time taken for an injected bolus to travel between a proximal position in the vessel, and a distal position in the vessel. Whilst guidelines clearly specify the location of the proximal position as the ostium of the vessel under investigation, the guidelines are less clear on the location of the distal position. Most of the current literature reports that the distal position should be chosen as a position in the "distal two-thirds" of the target vessel, as mentioned in a document by Kobayashi, Y., and Fearon, W. F., entitled "Invasive coronary microcirculation assessment - Current status of Index of Microcirculatory Resistance", Circulation Journal, Official Journal of the Japanese Circulation Society, 2014; 78(5); pages 1021-1028.

A consequence of the imprecise definition of the location of the distal position in such measurements is that microcirculatory resistance values, such as the IMR, and which rely on the value of the transit time, are frequently subject to an error. This error arises firstly from the range of locations that fall within the specified "distal two thirds" of a target vessel, secondly from the difficulty in accurately determining the length of a target vessel, and thirdly from the fact that the length of such vessels varies significantly between subjects. The error thus appears as inter- and intra-user variations in computed microcirculatory resistance values, and can ultimately affect a clinical diagnosis of a vessel.

Consequently, there is a need to improve the computation of a microcirculatory resistance value for a vessel.

The measurement of the transit time that is used to compute the microcirculatory resistance value for a vessel, may also be subject to an error. Consequently, there may also be a need to provide a corrected transit time measurement for a vessel. It may also be useful to determine a transit time for a vessel for other reasons that are not connected to computing a microcirculatory resistance value. Thus, in general it may be useful to improve the determination of a transit time for a vessel.

The computation of the microcirculatory resistance value for a vessel, may also rely on the determination of a length of a portion of a vessel. This length may be measured from X-ray projection images. The measurement of this length from X-ray images may also be subject to an error. Consequently, there may also be a need to compute a length of a portion of a vessel from an X-ray projection image. It may also be useful to determine a length of a portion of a vessel for other reasons that are not connected to computing a microcirculatory resistance value. Thus, in general it may be useful to improve the determination of a length of a portion of a vessel from X-ray projection images.

Summary

According to a first aspect of the present disclosure, a system for providing a normalised microcirculatory resistance value for a vessel, is provided. The system comprises one or more processors configured to:

- compute a microcirculatory resistance value for the vessel based on a transit time taken for an injected bolus to travel between a proximal position in the vessel, and a distal position in the vessel; and
- divide the computed microcirculatory resistance value by a transit length representing a length of the vessel between the proximal position and the distal position, to provide the normalised microcirculatory resistance value.

By dividing the computed microcirculatory resistance value by the transit length, the resulting normalised microcirculatory resistance value, is made to depend on a transit velocity, rather than simply a transit time. The normalised microcirculatory resistance value is consequently less sensitive to the imprecisely-defined location of the distal position. Thus, the normalised microcirculatory resistance value provides a more reliable metric for a vessel.

According to a second aspect of the present disclosure, a system for correcting a transit time of a vessel, is provided. The transit time represents a time taken for an injected bolus to travel along a transit length of the vessel between a proximal position in the vessel at a proximal time, and a distal position in the vessel at a distal time, and at an average transit velocity defined as a ratio of the transit length to the transit time. This system comprises one or more processors configured to:

receive measured cardiac cycle data for a heart fluidically coupled to the vessel, the measured cardiac cycle data comprising a measured cardiac period, and a time of a signature of each of one or more cardiac states within the cardiac cycle;
- map the time of the one or more signatures, and the proximal time, and the distal time, to corresponding times within the measured cardiac period;
- receive reference fluid velocity data comprising a time-dependent reference fluid velocity curve representing a fluid velocity in the vessel over a reference cardiac cycle having a reference cardiac period;
- identify in the reference fluid velocity curve, a time of a reference signature corresponding to each of the one or more cardiac states in the measured cardiac cycle data;
- transform a time axis of the reference fluid velocity curve such that the reference cardiac period matches the measured cardiac period, and such that the time of the reference signature of each of the one or more cardiac states identified in the reference fluid velocity curve corresponds to the time of the signature of each of the one or more corresponding cardiac states in the measured cardiac cycle data;
transform an amplitude axis of the transformed reference fluid velocity curve to provide an amplitude-transformed reference fluid velocity curve such that an average velocity of the amplitude-transformed reference fluid velocity curve computed over a time interval between the proximal time and the distal time, corresponds to the average transit velocity; and
- provide a corrected transit time by multiplying the transit time by a ratio of the average transit velocity to the average velocity of the amplitude-transformed reference fluid velocity curve over a full cardiac cycle.

The result of these operations is to provide a corrected transit time that is independent of the portion of the cardiac cycle during which the transit time is measured. This obviates some of the existing workflow complications associated with repeatedly injecting a bolus into the vessel and averaging multiple transit time measurements.

According to a third aspect of the present disclosure, a system for computing a length of a portion of a vessel from X-ray projection image data generated by an X-ray imaging system, is provided. This system comprises one or more processors configured to:

- receive X-ray image data representing an X-ray projection image comprising the portion of the vessel;
- receive X-ray imaging system geometric data representing an orientation of the X-ray imaging system respective the portion of the vessel;
- determine a length scale factor for positions along a length of the portion of the vessel in the X-ray projection image, based on a matching between the portion of the vessel in the X-ray projection image, and a reference vessel in a reference projection image generated by projecting a 3D model of a reference vessel representing the vessel using the X-ray imaging system geometric data; and
- compute the length of the portion of the vessel by scaling the vessel represented in the X-ray projection image, with the length scale factor determined at the corresponding positions along the length of the vessel.

In so doing, an accurate length of the portion of the vessel may be computed. The computed length is accurate since it takes account of foreshortening.

Further aspects, features, and advantages of the present disclosure will become apparent from the following description of examples, which is made with reference to the accompanying drawings.

Brief Description of the Drawings

Fig. 1 is a schematic diagram illustrating a heart, and includes an example of a proximal position $Pos_a$ and a distal position $Pos_a$ in a vessel 110, in accordance with some aspects of the present disclosure.

Fig. 2 is a schematic diagram illustrating an example of a system 100 for providing a normalised microcirculatory resistance value for a vessel, in accordance with some aspects of the present disclosure.

Fig. 3 is a flowchart illustrating an example of a method of providing a normalised microcirculatory resistance value for a vessel, in accordance with some aspects of the present disclosure.

Fig. 4 is a schematic diagram illustrating an example of an injection of a contrast agent bolus 140 into a vessel 110, and a temporal sequence of angiographic images 130 representing a flow of the injected bolus 140 through the vessel 110, in accordance with some aspects of the present disclosure.

Fig. 5 is a schematic diagram illustrating an example of a temporal sequence of angiographic images 130 representing a flow of an injected contrast agent bolus through the vessel 110, in accordance with some aspects of the present disclosure.

Fig. 6 is a schematic diagram illustrating an earlier image and a later image in temporal sequence of angiographic images 130, in accordance with some aspects of the present disclosure.

Fig. 7 is a schematic diagram illustrating a mapping of a proximal position $Pos_a$ from an earlier image, at time $T_0$, to a mapped position $Pos'_a$ in a later image, at time $T_1$, in a temporal sequence of angiographic images 130, in accordance with some aspects of the present disclosure.

Fig. 8 is a schematic diagram illustrating an angiographic image 130' including a vessel tree 150 and a vessel 110, in accordance with some aspects of the present disclosure.

Fig. 9 is a schematic diagram illustrating an example of a time-intensity curve 160 for a vessel tree, in accordance with some aspects of the present disclosure.

Fig. 10 is a schematic diagram illustrating an angiographic image 130' including a vessel tree 150 and an example of a longest minimal path $C^*(P_{ref},P^*_d)$ of vessel 110, in accordance with some aspects of the present disclosure.

Fig. 11 is a schematic diagram illustrating operations relating to a first example of correcting a transit time of a vessel, in accordance with some aspects of the present disclosure.

Fig. 12 is a schematic diagram illustrating operations relating to a second example of correcting a transit time of a vessel, in accordance with some aspects of the present disclosure.

Fig. 13 is a schematic diagram illustrating a 3D model of a reference vessel 170, in accordance with some aspects of the present disclosure.

Fig. 14 is a schematic diagram illustrating a projected 3D model of a reference vessel 170' on a virtual plane 180 of a detector of an X-ray imaging system, from a perspective of an X-ray source 190 of the X-ray imaging system, in accordance with some aspects of the present disclosure.

Fig. 15 is a schematic diagram illustrating X-ray imaging system geometric data representing an orientation of an X-ray imaging system 220 respective a portion of a vessel 110, including a rotational angle $\alpha$ of a central ray of the X-ray imaging system 220 around a longitudinal axis of a subject 230, in accordance with some aspects of the present disclosure.

Fig. 16 is a schematic diagram illustrating X-ray imaging system geometric data representing an orientation of an X-ray imaging system 220 respective the portion of the vessel 110, including a tilt angle $\beta$ of a central ray of the X-ray imaging system 220 with respect to a cranial-caudal axis of the subject 230, in accordance with some aspects of the present disclosure.

Fig. 17 is a flowchart illustrating an example of a method of correcting a transit time of a vessel, in accordance with some aspects of the present disclosure.

Fig. 18 is a flowchart illustrating an example of a method of computing a length of a portion of a vessel from X-ray projection image data generated by an X-ray imaging system, in accordance with some aspects of the present disclosure.

Detailed Description

Examples of the present disclosure are provided with reference to the following description and figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. It is also to be appreciated that features described in relation to one aspect may also be used in another aspect, and that all features are not necessarily duplicated in each aspect for the sake of brevity. For instance, features described in relation to providing a normalised microcirculatory resistance value for a vessel, features described in relation to correcting a transit time $T_T$ of a vessel, and features described in relation to computing a length of a portion of a vessel from X-ray projection image data, may be used in combination. Moreover, features described in relation to a system, may be implemented in a computer implemented method, and in a computer program product, in a corresponding manner.

In the following description, reference is made to various systems for determining vessel parameters. The vessel parameters that are determined include a microcirculatory resistance value for a vessel, a transit time of a vessel, and a length of a portion of a vessel. In some examples, vessel parameters are determined for a coronary vessel. For example, reference is made to an example of a vessel in the form of the left coronary artery. However, it is to be appreciated that this serves only as an example. The systems may be used to determine parameters in coronary vessels, as well as in vessels that are found elsewhere in the body. More generally, the systems may be used to determine parameters of vessels of the vasculature. The systems may be used to determine parameters in arteries and veins in the vasculature, including for example the iliac artery, the femoral artery, the popliteal artery, the tibial artery, the pulmonary arteries, systemic arteries.

Reference is also made herein to examples wherein angiographic data is used to determine vessel parameters. In this respect it is to be appreciated that the angiographic data may be generated by various types of X-ray imaging systems. By way of an example, the X-ray image data may be generated by the Philips Azurion 7 X-ray imaging system marketed by Philips Healthcare, Best, The Netherlands. The. angiographic data may in general be generated by a projection X-ray imaging system that generates 2D X-ray images, or by a volumetric X-ray imaging system that generates volumetric, i.e. 3D X-ray images. Projection X-ray imaging systems typically include a support arm such as a so-called "C-arm", or an "O-arm", that supports an X-ray source-detector arrangement. Projection X-ray imaging systems may alternatively include a support arm with a different shape to these examples. Projection X-ray imaging systems typically generate 2D X-ray images with the support arm held in a static position with respect to an imaging region during the acquisition of image data. By contrast, volumetric X-ray imaging systems typically generate image data whilst rotating, or stepping, an X-ray source-detector arrangement around an imaging region, and subsequently reconstruct the image data obtained from multiple rotational angles into a volumetric image. Examples of volumetric X-ray imaging systems include computed tomography "CT" imaging systems, cone beam CT "CBCT" imaging systems, and spectral CT imaging systems.

It is noted that the methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray™ and DVD.

As mentioned above, the present disclosure relates generally to the determination of vessel parameters. A first aspect of the present disclosure relates to a system for providing a normalised microcirculatory resistance value for a vessel. Microcirculatory resistance values, such as the Index of Microcirculatory Resistance, or "IMR", are typically computed for a vessel based on the measurement of a transit time taken for an injected bolus to travel between a proximal position in a vessel, and a distal position in the vessel. For example, the IMR is defined by the Equation:

$$IMR = P_d \cdot T_T \qquad\qquad \text{Equation 1}$$

wherein $P_d$ represents the distal pressure at the distal position in the vessel, and $T_T$ represents the transit time. The IMR is typically calculated with Equation 1 using the time-averaged value of the distal pressure $P_d$ over a cardiac cycle at maximal hyperemia.

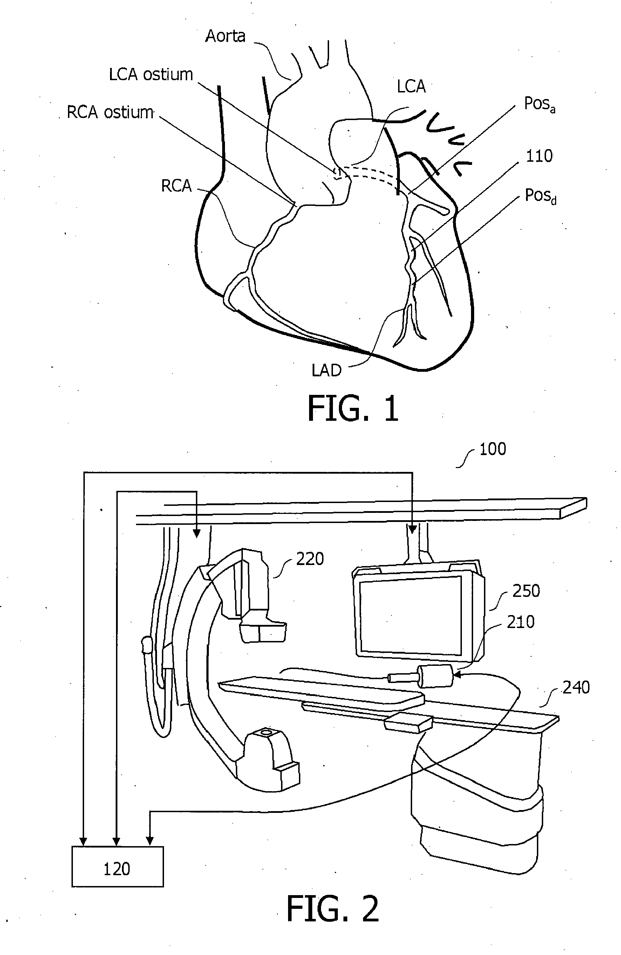

Fig. 1 is a schematic diagram illustrating a heart, and includes an example of a proximal position $Pos_a$ and a distal position $Pos_d$ in a vessel 110, in accordance with some aspects of the present disclosure. An IMR measurement is typically made for one of the main arteries, such as the Left Anterior Descending "LAD" artery, the Left Circumflex artery "LCX", or the Right Coronary Artery "RCA" illustrated in Fig. 1. By way of an example, a value for the IMR may be determined for the LAD artery illustrated in Fig. 1, and which is a branch of the left coronary artery tree, LCA. The example proximal position $Pos_a$ and distal position $Pos_d$ in Fig. 1 are illustrated for the LAD artery, $Pos_a$ being close to the ostium of the LAD artery, and $Pos_b$ being within the lower two thirds of the LAD artery.

If a vessel is subject to severe epicardial stenosis, an alternative definition for the IMR has been proposed, and wherein the IMR is computed using the Equation:

$$IMR = P_d \cdot T_T \cdot \left(\frac{P_d - P_w}{P_a - P_w}\right) \qquad\qquad \text{Equation 2}$$

In Equation 2, the additional term $P_a$ represents the proximal pressure at the proximal position in the vessel, and the additional term $P_w$ represents the coronary wedge pressure, i.e. the pressure at a distal position to the stenosis when the vessel is occluded by an inflated balloon. The IMR is typically calculated with Equation 2 using the time-averaged value of the proximal pressure Pa over a cardiac cycle at maximal hyperemia, using the time-averaged value of the distal pressure $P_d$ over a cardiac cycle at maximal hyperemia, and using the time-averaged value of the pressure over a cardiac cycle at the distal position $Pos_d$ in the vessel when the vessel is occluded by an inflated balloon the wedge pressure $P_w$. Alternative equations for computing the IMR may also be used, and these likewise depend on the transit time $T_T$.

The IMR is often measured using the thermodilution technique wherein an intraluminal pressure sensor and two temperature sensors are inserted into the vessel. The pressure sensor measures the intraluminal pressure, and the temperature sensors are arranged at separated positions in the vessel in order to measure the transit time $T_T$ taken by a temperature transition induced on the blood by an injected room-temperature saline water bolus to travel between the two temperature sensors. In the thermodilution technique, the IMR is often calculated using a transit time $T_T$ that is computed as an average of multiple individual transit time measurements. More recently, an alternative angiographic technique has been investigated to determine the IMR. In this technique, the positions of a front of an injected contrast agent bolus are detected in angiographic images and used to determine the transit time $T_T$ taken by the front to travel between a proximal and a distal position in the vessel. A front is identifiable in angiographic images based on a change in intensity of the angiographic image along a vessel.

Irrespective of which technique is used to determine the transit time, $T_T$, the transit time represents the time taken for an injected bolus to travel between a proximal position in a vessel, and a distal position in the vessel. Whilst guidelines clearly specify the location of the proximal position as the ostium of the vessel under investigation, the guidelines are less clear on the location of the distal position. Most of the current literature reports that the distal position should be chosen as a position in the "distal two-thirds" of the target vessel, as mentioned in the document by Kobayashi, Y., and Fearon, W. F., entitled "Invasive coronary microcirculation assessment - Current status of Index of Microcirculatory Resistance", Circulation Journal, Official Journal of the Japanese Circulation Society, 2014; 78(5); pages 1021-1028.

A consequence of the imprecise definition of the location of the distal position in such measurements is that microcirculatory resistance values, such as the IMR, and which rely on the value of the transit time, are frequently subject to an error. This error arises firstly from the range of locations that fall within the specified "distal two thirds" of a target vessel, secondly from the difficulty in accurately determining the length of a target vessel, and thirdly from the fact that the length of such vessels varies significantly between subjects. The error thus appears as inter- and intra-user variations in computed microcirculatory resistance values, and can ultimately affect a clinical diagnosis of a vessel.

The inventors have determined that an improvement to a microcirculatory resistance value for a vessel, may be provided by normalising the microcirculatory resistance value with a transit length $d_T$ representing a length of the vessel between the proximal position $Pos_a$ and the distal position $Pos_d$. In accordance with a first aspect of the present disclosure, a system 100 for providing a normalised microcirculatory resistance value for a vessel 110, is provided. The system comprises one or more processors 120 configured to:

- compute S110 a microcirculatory resistance value for the vessel 110 based on a transit time $T_T$ taken for an injected bolus to travel between a proximal position $Pos_a$ in the vessel, and a distal position $Pos_d$ in the vessel; and
- divide S120 the computed microcirculatory resistance value by a transit length $d_T$ representing a length of the vessel between the proximal position $Pos_a$ and the distal position $Pos_d$, to provide the normalised microcirculatory resistance value.

By dividing the computed microcirculatory resistance value by the transit length $d_T$, the resulting normalised microcirculatory resistance value, is made to depend on a transit velocity, rather than simply a transit time. The normalised microcirculatory resistance value is consequently less sensitive to the imprecisely-defined location of the distal position. Thus, the normalised microcirculatory resistance value provides a more reliable metric for a vessel. This is especially important in view of the role of the microcirculatory resistance value, which among other indications, inform a clinician who may perform a clinical diagnosis on the vessel.

Computing the normalised microcirculatory resistance value in this manner, also permits its comparison with microcirculatory resistance values from previous clinical studies. Specifically, the normalised microcirculatory resistance values can be computed without requiring a change to a clinical site's interpretation of the imprecisely-defined location of the distal position. The data that is used to calculate the normalised microcirculatory resistance value, may therefore also be used to calculate the known microcirculatory resistance value. This allows a clinician to compare the normalised microcirculatory resistance values, with microcirculatory resistance values from historic clinical investigations. By providing continuity between the new normalised microcirculatory resistance value, and existing microcirculatory resistance values, the more reliable metric may be used to build-upon the existing clinical knowledge for the microcirculatory resistance value.

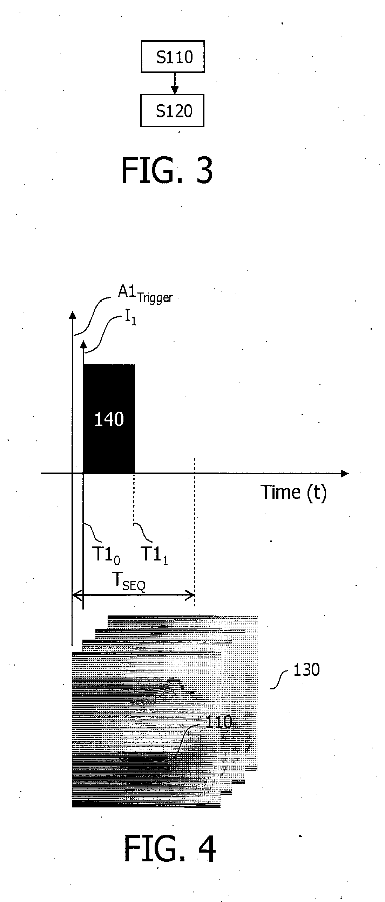

The system 100 is described with reference to Fig. 2, which is a schematic diagram illustrating an example of a system 100 for providing a normalised microcirculatory resistance value for a vessel, in accordance with some aspects of the present disclosure. The operations performed by the system are also described with reference to Fig. 3, which is a flowchart illustrating an example of a method of providing a normalised microcirculatory resistance value for a vessel, in accordance with some aspects of the present disclosure. Operations performed in accordance with the system 100 illustrated in Fig. 2 may also be performed by the method illustrated in Fig. 3, and vice versa.

With reference to Fig. 2 and Fig. 3, in the operation S110, a microcirculatory resistance value for the vessel 110 is computed based on a transit time $T_T$ taken for an injected bolus to travel between a proximal position $Pos_a$ in the vessel, and a distal position $Pos_d$ in the vessel.

The vessel for which the microcirculatory resistance value is computed in the operation S110 may for example be the LAD vessel in the left coronary artery, as illustrated in Fig. 1. However, as mentioned above, the vessel may alternatively be another vessel in the vasculature.

In general, the proximal position $Pos_a$ and the distal position $Pos_d$ that are used to compute the transit time $T_T$ in the operation S110 are defined with respect to the ostium of the relevant vessel. Thus in the example illustrated in Fig. 1, the proximal position $Pos_a$ is relatively closer to the LAD ostium, and the distal position $Pos_d$ is relatively further from the LAD ostium. The proximal position and distal positions also refer to positions with respect to conventional blood flow in the vessel; the proximal position being relatively upstream with respect to conventional blood flow in the vessel, and the distal position being relatively downstream with respect to conventional blood flow in the vessel. When calculating flow parameters, such as the IMR, the distal position $Pos_d$ may refer more specifically to a position in the "distal two-thirds" of the relevant vessel, as mentioned in the document by Kobayashi, Y., and Fearon, W. F., entitled "Invasive coronary microcirculation assessment - Current status of Index of Microcirculatory Resistance", Circulation Journal, Official Journal of the Japanese Circulation Society, 2014; 785; pages 1021-1028. A distal position that is the most distal position in the relevant vessel in an angiographic image, is sometimes used to calculate such flow parameters.

The transit time $T_T$ that is computed in the operation S 110 may be determined using various techniques. In one example, the transit time is determined using the thermodilution technique outlined above. In another example, the transit time is determined using an angiographic technique outlined above. Other techniques may also be used to determine the transit time of an injected bolus.

In the thermodilution technique, an intraluminal pressure sensor and two temperature sensors are inserted into the vessel. The pressure sensor may form part of a so-called "pressure wire", for example. The temperature sensors may be arranged on the pressure wire, or they may be included on one or more further intraluminal devices. The pressure sensor measures the intraluminal pressure, and the temperature sensors are arranged at separated positions along the vessel. A first of the two temperature sensors, a proximal temperature sensor, is arranged at a proximal position Posa in the vessel, and a second of the two temperature sensors is arranged at a distal position $Pos_d$ in the vessel. The pressure sensor and the temperature sensors are typically inserted under X-ray imaging guidance, and their positions adjusted such that the proximal and distal temperature sensors are arranged at the desired proximal and distal positions in the vessel. In-use, a room-temperature saline water bolus is injected into the vessel, and the temperature sensors measure the transit time $T_T$ taken by a temperature transition induced on the blood by the bolus to travel between the

proximal position $Pos_a$ and the distal position $Pos_d$.

An alternative to measuring the transit time $T_T$ using the thermodilution technique, is to measure the transit time using angiographic images. The angiographic images may be generated using the X-ray imaging system 220 illustrated in Fig. 2. In this technique, a contrast agent bolus is injected into the vessel, and the positions of a front of an injected contrast agent bolus are detected in a temporal sequence of angiographic images. Depending on the vessel of interest, in some examples, the angiographic images may be coronary angiographic images. The proximal position $Pos_a$ is defined as a position of the detected front in an earlier X-ray image in the temporal sequence 130, and the distal position $Pos_d$ is defined as a position of the detected front in a later X-ray image in the temporal sequence 130. The transit time $T_T$ corresponds to a time difference between the earlier X-ray image and the later X-ray image. The contrast agent may include Iodine, or a Lanthanide such as Gadolinium, or indeed another substance that provides visibility of a flow in the vessel into which the contrast agent is injected.

In the thermodilution technique, and likewise in the angiographic technique, the respective saline, or contrast agent bolus may be injected from a syringe that is fluidically coupled to the vessel 110. The syringe may be operated manually, or using an injector, such as the injector 210 illustrated in Fig. 1. The injector 210 includes a syringe and a syringe driver that is configured to apply pressure to the syringe. The syringe contains the saline, or the contrast agent, and is fluidically coupled to the vessel via an injection catheter such that when the syringe driver applies pressure to the syringe, the syringe injects the saline, or the contrast agent, through the injection catheter and into the vessel 210. The injector 210 may be operated manually, or automatically, in order to deliver a desired dose of saline, or contrast agent, to the vessel. An example of a suitable injector for this purpose is the Medrad Mark 7 Arterion ™ Injection System marketed by Medrad Europe, B.V., Beek, The Netherlands. This, however, is mentioned purely by way of an example.

Various microcirculatory resistance values may be calculated using the transit time $T_T$, in the operation S110. In some examples, the microcirculatory resistance value is determined by multiplying the transit time $T_T$ by a distal intraluminal pressure value $P_d$, the distal intraluminal pressure value $P_d$ representing a pressure in the vessel at the distal position $Pos_d$ in the vessel. The microcirculatory resistance value may for example be an index of microcirculatory resistance, IMR, value, although other resistance values that are based on the transit time $T_T$ may also be computed. The IMR value may be computed using Equation 1, or Equation 2, defined above, although other equations may also be used to compute the IMR value.

In one example, the one or more processors 120 are further configured to multiply the computed microcirculatory resistance value by a reference vessel length do, to provide the normalised microcirculatory resistance value. The reference vessel length $d_0$ may for example be a population-averaged length of the vessel. In this example, a normalised value for the IMR, $IMR_{d0}$, may be calculated from the IMR using Equation 3.

$$IMR_{d_0} = IMR \cdot \frac{d_0}{d_T} \qquad \qquad \text{Equation 3}$$

Substituting the IMR from Equation 1 into Equation 3, results in:

$$IMR_{d_0} = P_d \cdot T_T \cdot \frac{d_0}{d_T} = P_d \cdot \frac{d_0}{v_T} = HMR \cdot d_0 \qquad \qquad \text{Equation 4}$$

wherein HMR is the Hyperaemic Microvascular Resistance index. Thus, further multiplying the computed normalised microcirculatory resistance value by the reference vessel length $d_0$ in this manner, results in a flow parameter that is scaled to the population average. The role of the multiplication by do is also to scale the normalised IMR to the current range of non-normalized IMR. In such a way, the obtained values are easily understood by physicians or cardiologists accustomed to the non-normalized values. For instance, they can use the same cut-value to decide whether a patient should be considered as suffering from a microvascular condition. A typical value for non-acute patient is $IMR_{cut}$ = 25. Above this value the index of microvasculature resistance is deemed high, leading to a positive diagnostic of microvascular disease. If $d_0$ is properly chosen, the same cut-value can be used for $IMR_{d0}$.

A value for the distal pressure $P_d$ used in Equation 1 - Equation 4 may be measured using an intraluminal pressure sensor, or it may be estimated. The distal pressure $P_d$ may for example be measured using an intraluminal pressure sensor that is disposed at the distal position. Alternatively, a value for the distal pressure $P_d$ may be estimated based on a measurement of the proximal pressure Pa. The proximal pressure Pa may be measured using an intraluminal pressure sensor disposed at the proximal position, or it may be inferred from an extracorporeal pressure sensor that measures a pressure of the fluid in the syringe that injects the contrast agent.

Injectors, such as the injector 210 illustrated in Fig. 1 often include such an extracorporeal pressure sensor in order to monitor the pressure in. the vessel during the injection of a bolus. Since the extracorporeal pressure sensor is fluidically coupled to the vessel, and since fluid is incompressible, the extra-corporeal pressure sensor provides a measurement of the intraluminal pressure at the distal end of the injection catheter from an extracorporeal position. Since the distal end of the injection catheter is typically close to the proximal position $Pos_d$, the extra-corporeal pressure sensor provides a reliable measurement of the proximal pressure $P_a$.

As mentioned above, the value of the distal pressure $P_d$ may also be estimated. The distal pressure $P_d$ may be estimated from the measured proximal pressure $P_a$ using a geometric model of the vessel. The geometric model may be provided by segmenting one or more angiographic images of the vessel. Various image segmentation techniques are known for this purpose, including thresholding, template matching, active contour modelling, model-based segmentation, neural networks, e.g., U-Nets, and so forth. Angiographic images representing one or more views of the vessel may be segmented in order to provide geometric measurements of the vessel 110 that are used to construct a model of the vessel with which to model fluid flow. By way of an example, the vessel may be represented by a 3D centerline with elliptic cross-sections along a length of the 3D centerline. A ID, or a 3D hemodynamic model, which may be based on Navier-Stokes partial differential equations, may then be used with the geometric model to determine the fluid flow in the vessel, and ultimately to estimate the distal pressure $P_d$. The 3D haemodynamic model may be solved in 3D space using Computational Fluid Dynamics, "CFD" techniques.

A value for the wedge pressure $P_w$ in Equation 2 may be measured in a similar manner to the distal pressure $P_d$. Thus, the value of the wedge pressure $P_w$ may be measured using an intraluminal pressure sensor. Alternatively, the wedge pressure $P_w$ may be estimated using a geometric model in a similar manner to the estimated distal pressure $P_d$.

With continued reference to Fig. 3, in the operation S120, the microcirculatory resistance value that is computed in the operation S110, is then divided by a transit length $d_T$ representing a length of the vessel between the proximal position $Pos_a$ and the distal position $Pos_d$, to provide the normalised microcirculatory resistance value.

Various techniques may be used to determine the transit length $d_T$, that is used in the operation S120. In one technique, the transit time $T_T$ is measured using the thermodilution technique, and the transit length $d_T$ is be determined from a known separation between proximal temperature sensor and the distal temperature sensor, that are used to measure the transit time $T_T$. In this technique, the one or more processors 120 receive intraluminal sensor data comprising temperature data, and the transit time $T_T$, is determined from the received intraluminal sensor data. Moreover, the intraluminal sensor data is provided by an intraluminal device comprising a proximal temperature sensor and a distal temperature sensor, and the one or more processors 120 are further configured to determine the transit time $T_T$ based on the time taken for a temperature transition induced by an injected bolus to travel between the proximal temperature sensor in the vessel 110, and the distal temperature sensor in the vessel. The transit length $d_T$ corresponds to a length of the intraluminal device between the proximal temperature sensor and the distal temperature sensor. In this technique, the positions of the proximal and distal temperature sensors may be fixed, or they may be movable to known positions, and thus the transit length $d_T$ may be known. For example, the proximal and distal temperature sensors may be disposed at known positions on the intraluminal device. Alternatively, the position of one or more of the temperature sensors may be adjustable to a known location, or a location that is calculable. For example, a temperature sensor may be translateable along a length of the intraluminal device to one or more discrete positions. Alternatively, a length scale may be provided at a proximal end of the intraluminal device and used to measure an amount of translation of a temperature sensor along a length of the intraluminal device, and thereby measure a separation between the temperature sensors. Alternatively, fiducial markers may be disposed in known locations on the intraluminal device and X-ray imaging used to determine the positions of the temperature sensors in relation to the fiducial markers, and thereby determine a separation between the temperature sensors.

In another technique, X-ray angiographic image data is used to determine the transit time $T_T$ and an estimate of the transit length $d_T$. The angiographic image data may be generated using the X-ray imaging system 220 illustrated in Fig. 2. In this technique, the processor(s) 120 receive X-ray angiographic image data comprising a temporal sequence of images 130 representing a flow of the injected bolus through the vessel 110; and the one or more processors 120 are configured to:

- analyse the X-ray image data to determine the transit time $T_T$ and an estimate of the transit length $d_T$.

This technique is described in more detail with reference to Fig. 4 - Fig. 9. Fig. 4 is a schematic diagram illustrating an example of an injection of a contrast agent bolus 140 into a vessel 110, and a temporal sequence of angiographic images 130 representing a flow of the injected bolus 140 through the vessel 110, in accordance with some aspects of the present disclosure. The upper portion of Fig. 4 illustrates various timing signals that represent the injection of the contrast agent bolus 140 into the vessel 110. In the upper portion of Fig. 4, the contrast agent bolus 140 is illustrated by means of a dark-shaded pulse that represents the contrast agent injection rate on the vertical axis, over time, "Time (t)", on the horizontal axis. The injection begins at a time $T1_0$, and ends at a time $T1_1$. The contrast agent injection may be initiated in response to an injection trigger signal $I_1$, which is also illustrated in Fig. 3. The injection trigger signal $I_1$ may be generated manually, or by the processor 120. The contrast agent injection rate in this example is constant during

the period from $T1_0$ to $T1_1$. The contrast agent injection rate may alternatively vary over time.

The lower portion of Fig. 4 illustrates a temporal sequence of angiographic images 130. The acquisition of the temporal sequence may be initiated by an imaging trigger signal $A1_{Trigger}$, as illustrated in the upper portion of Fig. 4. The imaging trigger signal $A1_{Trigger}$ may be generated manually, or by the processor 120. By generating the imaging trigger signal $A1_{Trigger}$ and/or the injection trigger signal $I_1$ using the processor 120, the system 100 may provide reliable synchronisation of the generation of the temporal sequence 130 with the contrast agent injection 140. This may obviate potential timing errors associated with manually triggering these operations, and which might otherwise result in the need to repeat the contrast agent injection 130. The imaging trigger signal $A1_{Trigger}$ may be generated prior to the start of the injection of the contrast agent. The temporal sequence may also have a duration $T_{SEQ}$ that overlaps with the duration of the injection of the contrast agent during the period from $T1_0$ to $T1_1$, such that the temporal sequence captures a flow of the injected contrast agent bolus through the vessel 110.

In one example of this angiographic technique, the proximal position $Pos_a$) and the distal position $Pos_d$ in the vessel 110, may also be identified in the X-ray image data. In this example, the injected bolus comprises an injected contrast agent bolus 140, and the processor(s) 120 are configured to:

- identify the proximal position $Pos_a$ and the distal position $Pos_d$ in the vessel 110 in the X-ray image data;
- determine the transit time $T_T$ based on the time taken for the injected contrast agent bolus 140 to travel between the identified proximal position $Pos_a$ in the vessel, and the identified distal position $Pos_d$ in the vessel, in the X-ray image data; and
- compute the estimate of the transit length $d_T$ based on a length of the vessel 110 between the proximal position $Pos_a$ and the distal position $Pos_d$, in the X-ray image data.

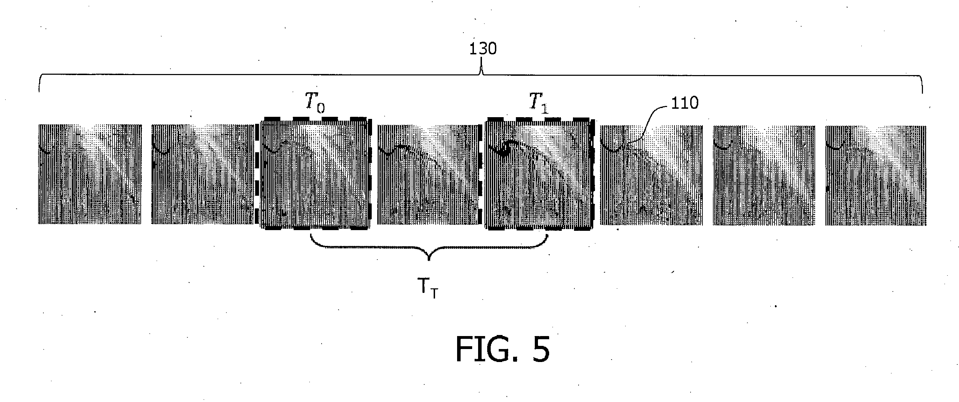

This example is described with reference to Fig. 5, which is a schematic diagram illustrating an example of a temporal sequence of angiographic images 130 representing a flow of an injected contrast agent bolus through the vessel 110, in accordance with some aspects of the present disclosure. The temporal sequence illustrated in Fig. 5 maybe generated subsequent to the injection described in relation to Fig. 4. In Fig. 5, the injected contrast agent can be seen to progress through the vessel 110 over time. At time $T_0$, a front of the contrast agent is at a proximal position near to the ostium of the LAD. Two image frames later, at time $T_1$, a front of the contrast agent is at a distal position in the vessel 110. The positions of the front may be detected in the angiographic images 130 using known image processing techniques. By way of an example, the proximal position $Pos_a$ and the distal position $Pos_d$ in the in the vessel may be detected automatically by detecting the position of the front, and defining the position as a proximal position or a distal position if the front is within a predetermined distance of the relevant ostium, or within the distal two thirds of the vessel, respectively. The proximal position $Pos_a$ and the distal position $Pos_d$ in the vessel may also be identified based on user input. The user input may be received from a user input device such as a keyboard, a mouse, a touchscreen, and so forth. The user input may specify an image frame, and a position of the front in the image frame, that corresponds to each of the proximal position $Pos_a$ and the distal position $Pos_d$. With reference to Fig. 5, the transit time $T_T$ may then be computed as the time difference between the time $T_0$ and the time $T_1$. An estimate of the transit length $d_T$ is then calculated based on a length of the vessel 110 between the proximal position $Pos_a$ and the distal position $Pos_d$, in the X-ray image data.

In one example, the length of the vessel 110 between the proximal position $Pos_a$ and the distal position $Pos_d$, is determined by mapping the proximal position and the distal position to a common image. In this example, the proximal position $Pos_a$ corresponds to a position of a detected front of the injected contrast agent bolus 140 in the vessel 110 in an earlier X-ray image in the temporal sequence 130, and the distal position $Pos_d$ corresponds to a position of the detected front in a later X-ray image in the temporal sequence 130. Moreover, the transit time $T_T$ corresponds to a time difference between the earlier X-ray image and the later X-ray image; and the estimate of the transit length $d_T$ is computed by:

- mapping the proximal position $Pos_a$ from the earlier image to the later image to provide a mapped proximal position $Pos'_a$ in the later image, or by mapping the distal position $Pos_d$ from the later image to the earlier image to provide a mapped distal position $Pos'_d$ in the earlier image; and
- determining a length of the vessel between the mapped proximal position $Pos'_a$ and the distal position $Pos_d$ in the later image, or between the proximal position $Pos_a$ and the mapped distal position $Pos'_d$ in the earlier image, respectively.

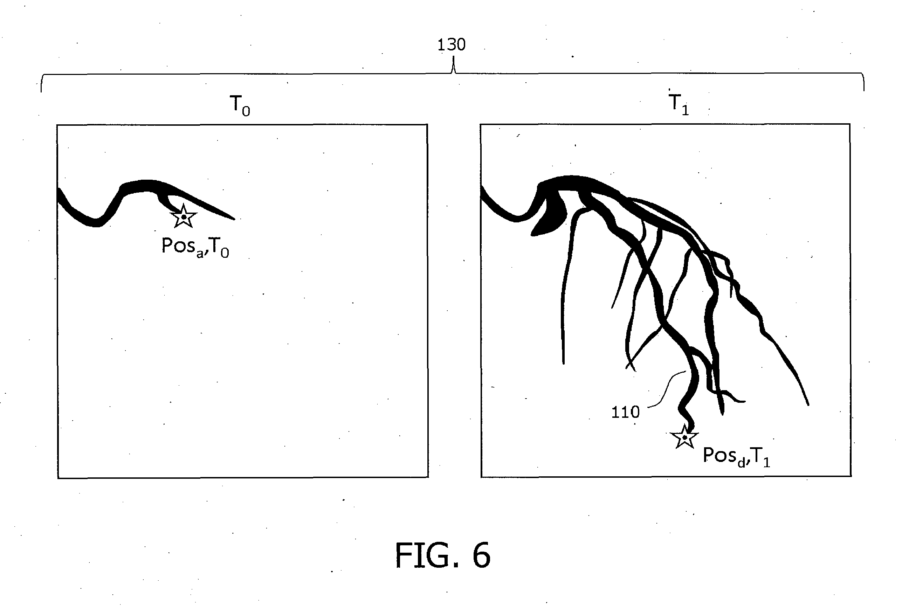

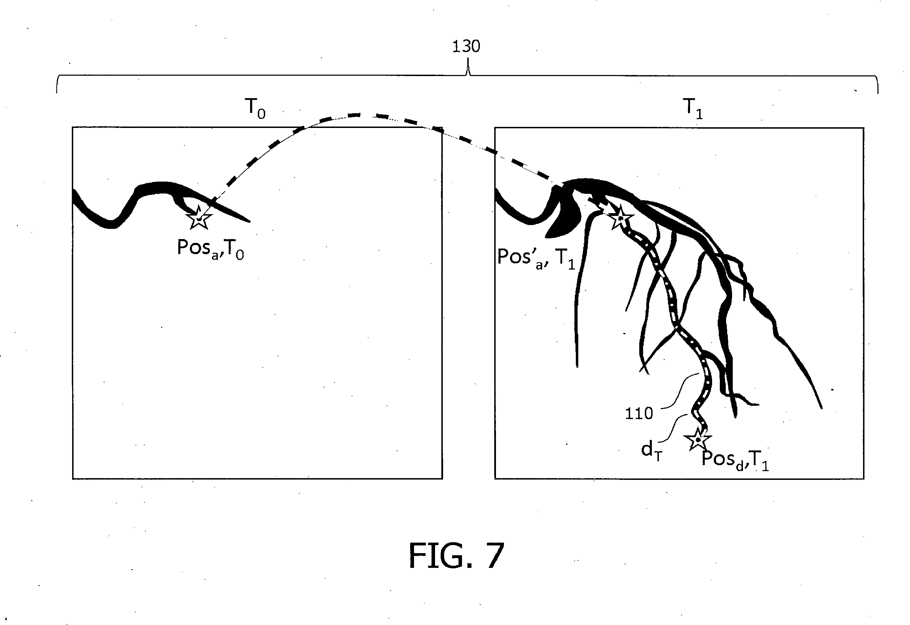

This example is described with reference to Fig. 6, which is a schematic diagram illustrating an earlier image and a later image in temporal sequence of angiographic images 130, in accordance with some aspects of the present disclosure, and with reference to Fig. 7, which is a schematic diagram illustrating a mapping of a proximal position $Pos_a$ from an earlier image, at time $T_0$, to a mapped position $Pos'_a$ in a later image, at time $T_1$, in a temporal sequence of angiographic images 130, in accordance with some aspects of the present disclosure.

The left-hand portion of Fig. 6 illustrates an earlier X-ray image in a temporal sequence 130, and which is generated at a time $T_0$. This image corresponds to the image at time $T_0$ in Fig. 5. In this image in Fig. 6, a front of the contrast agent is at a proximal position $Pos_a$ in the vessel 110. The right-hand portion of Fig. 6 illustrates a later X-ray image in the temporal sequence 130, and which is generated at a time $T_1$. This image corresponds to the image at time $T_1$ in Fig. 5. In this image in Fig. 6, a front of the contrast agent is at a proximal position $Pos_d$ in the vessel 110. The transit time $T_T$ corresponds to a time difference between the earlier X-ray image at time $T_0$, and the later X-ray image at time $T_1$ in Fig. 6.

In Fig. 7, the mapping operation is illustrated by way of the dashed line between the proximal position $Pos_a$ in the earlier image at time $T_0$ and the corresponding mapped position $Pos'_a$ in the later image a time $T_1$. This mapping may be performed using various techniques. In one example technique, the two images may be registered to one another. This technique may provide reliable results in the presence of a small amount of motion between the two images. In another example technique, a centerline mapping approach may be used wherein a centerline is identified and tracked in each of the earlier and later images. This example technique may be implemented by a path finding technique. By way of an example, a path finding algorithm, such as Fast-Marching, may be used repetitively to estimate the centerline of a vessel in the earlier image whilst being constrained by the resemblance (for instance in position, angulation, curvature) to the centerline of the vessel in a later image. The mapping operation described with reference to Fig. 7 may alternatively be performed in a similar manner by mapping the distal position $Pos_d$ from the later image to a mapped distal position $Pos'_d$ the earlier image.

Having performed the mapping as described above, the transit length $d_T$ is computed by calculating the length of the vessel 110 between the proximal position $Pos_a$ and the distal position $Pos_d$. This length may be determined from the angiographic image that includes the mapped position, i.e. the later image at time $T_1$ in the example illustrated in Fig. 7, and using a known calibration of the X-ray imaging system 220. The calibration may provide a scaling between angiographic image pixels and a real-world dimension, such as millimetres or centimetres per pixel in the patient. Such a calibration may be known for the current geometry of the X-ray imaging system. It may also be computed based on the geometry of the X-ray imaging system 220 using parameters such as the relative separation between the X-ray source, vessel 110, and X-ray detector, and a linear dimension of the X-ray detector. Alternatively, such a calibration may be determined based on the known dimensions of a feature that is included in the angiographic images.

As mentioned above, various positions may be selected as the distal position $Pos_d$, in the vessel 110. The distal position $Pos_d$ may for example correspond to:

- the most distal position in the vessel 110 in the X-ray image data away from the proximal position; or
- a position in the distal two-thirds of the vessel in the X-ray image data;
- a position in the vessel in the X-ray image data providing an estimate of the transit length $d_T$ that exceeds a reference transit length; or
- the most distal identifiable position in the vessel providing an estimate of the transit length $d_T$ that is not locally foreshortened above a predetermined reference foreshortening value.

In another example, the transit time $T_T$ is determined from a temporal sequence of angiographic images that represents a vessel tree. In this example, the vessel 110 forms part of a vessel tree 150, and the temporal sequence of images 130 represents the vessel tree, and further represents a flow of the injected contrast agent bolus 140 through the vessel tree. In this example, the processor(s) 120 are configured to:

- generate a time-intensity curve 160 for the vessel tree from the temporal sequence of images 130; and
- calculate the transit time $T_T$ from the time-intensity curve, the transit time being defined by a difference between a first point in time $T_{0a}$ at which the bolus 140 enters a portion of the vessel tree, and a second point in time $T_{1a}$ at which the bolus 140 saturates the vessel tree, in the corresponding images in the temporal sequence 130.

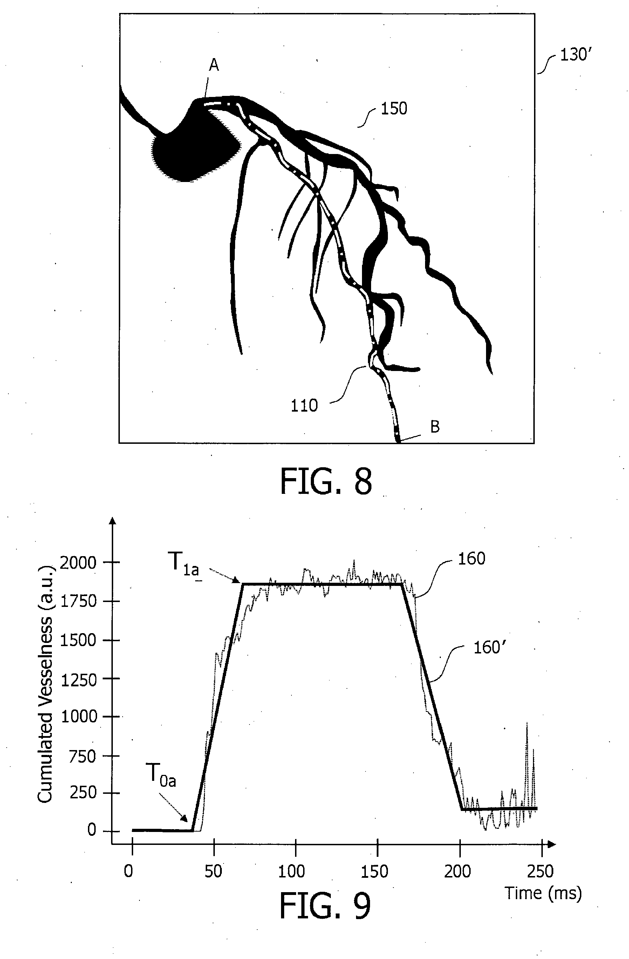

This example is described with reference to Fig. 8 and Fig. 9. Fig. 8 is a schematic diagram illustrating an angiographic image 130' including a vessel tree 150 and avessel 110, in accordance with some aspects of the present disclosure. In Fig. 8, the vessel 110 represents the LAD artery, and the vessel 110 forms part of the vessel tree 150. In the angiographic image 130' illustrated in Fig. 8, the contrast agent has saturated the vessel tree. Fig. 9 is a schematic diagram illustrating an example of a time-intensity curve 160 for a vessel tree, in accordance with some aspects of the present disclosure. In the time intensity curve illustrated in Fig. 9, the ordinate "Cumulated Vesselness (a.u.)" represents the intensity of the contrast agent in arbitrary units, and the abscissa "Time (ms)" represents time in milliseconds. The time intensity curve illustrated in Fig. 9 is generated by calculating, for each of multiple angiographic images in a temporal sequence 130, the cumulated values of a "vesselness" image corresponding to the angiographic image at the time of its acquisition. The pixels in the "vesselness" image represent the probability that the corresponding pixels in the angiographic image belongs to a target vessel. The cumulated values of each "vesselness" image are then calculated in order to provide the "Cumulated Vesselness" values that are plotted in Fig. 9. Computer vision approaches, for instance based on 2nd derivatives or Neural Networks approaches are well known techniques to compute such vesselness images. One suitable technique is described in a document by Iyer, K., et al., entitled "AngioNet: A Convolutional Neural Network for Vessel Segmentation in X-ray Angiography": medRxiv 2021.01.25.21250488; doi: https://doi.org/10.1101/2021.01.25.21250488.

In Fig. 9, at Time = 0 milliseconds, the Cumulated Vesselness value starts at a background level. At time $T_{0a}$, the bolus 140 enters the portion of the vessel tree represented in the angiographic images. The Cumulated Vesselness increases after time $T_{0a}$, until time $T_{1a}$, and at which time the bolus 140 saturates the vessel tree. The time $T_{1a}$ corresponds to the time of the angiographic image 130' illustrated in Fig. 8. Following the time $T_{1a}$, the vessel tree remains saturated with the contrast agent bolus, and thus the Cumulated Vesselness remains constant and at a maximum value, until wash-out starts at Time = approximately 160 milliseconds. During washout, the trailing edge of the contrast agent bolus passes through the vessel tree, and the Cumulated Vesselness decreases towards the background level again. After Time = approximately 200 milliseconds, the bolus has completely washed-out of the vessel tree represented in the angiographic images, and the Cumulated vesselness remains at the background level.

The time $T_{0a}$ at which the bolus 140 enters the portion of the vessel tree, and the time $T_{1a}$ at which the bolus 140 saturates the vessel tree, may be determined by analysing time intensity curve 160. The time $T_{0a}$ may represent the time at which the bolus enters the ostium of a vessel of interest. A threshold level may be used to detect the time $T_{0a}$ at which the time intensity curve first exceeds the threshold level. Similarly, the time $T_{1a}$ at which time the bolus 140 saturates the vessel tree may be determined by detecting the time at which the intensity first reaches a level that is within a predetermined range from the maximum intensity. The time $T_{1a}$ may for example correspond to the time at which the time intensity curve first reaches a level that is ninety percent of the maximum intensity. The transit time $T_T$ may then be calculated as the difference between the time $T_{0a}$ and the time $T_{1a}$.

The time intensity curve 160 illustrated in Fig. 9 can be seen to include some noise. In order to reduce the impact of the noise when analysing the time intensity curve, a fitting procedure may be used to fit a line to the time intensity curve 160. The times $T_{0a}$ and $T_{1a}$ may be determined from the fitted line. By way of an example, a straight-line fit 160' to the time intensity curve 160 is illustrated in Fig. 9. The times $T_{0a}$ and $T_{1a}$ may likewise be determined by analysing the straight-line fit 160' in a similar manner to the time intensity curve 160. Other lines, such as curves, may alternatively be fitted to the time intensity curve 160 in order to determine the times $T_{0a}$ and $T_{1a}$.

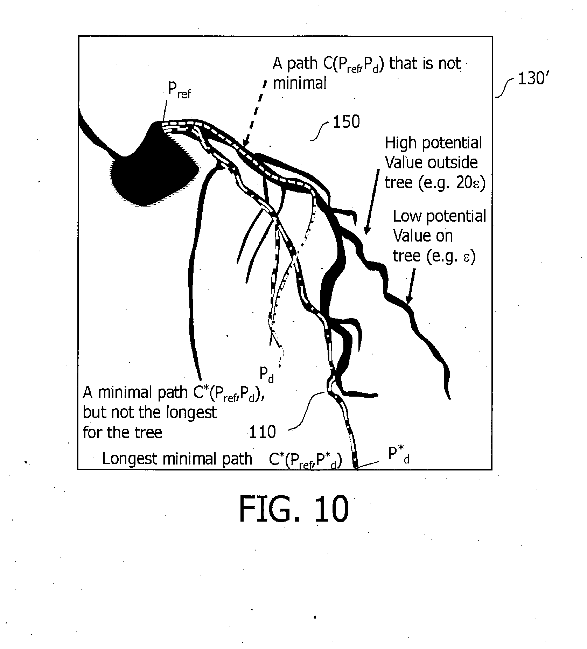

In this example wherein the transit time $T_T$ is determined from the vessel tree 150, a corresponding transit length $d_T$ may be provided by the length of the longest vessel in the vessel tree represented in the temporal sequence of angiographic images 130. In this regard, the longest vessel may be the longest identifiable vessel in the vessel tree. The longest identifiable vessel in the vessel tree may be estimated as the longest of the shortest paths of the tree between a reference point close to the ostium of the tree and any other point of the tree. This is illustrated with reference to Fig. 10, which is a schematic diagram illustrating an angiographic image 130' including a vessel tree 150 and an example of a longest minimal path $C^*(P_{ref}, P^*_d)$ of vessel 110, in accordance with some aspects of the present disclosure. A shortest path between the reference point and any point of the tree can be computed with a minimal path technique such as Fast-Marching. Starting from the reference point, for every point of the tree, the minimal path for that point is the one that leads to this point while covering the shortest geodesic distance. The geodesic distance over a path can be defined as the cumulated potential values along that path, where the potential value at any pixel is for instance a function of the vesselness. The higher the vesselness, the lower the potential value. By way of an example, the potential value is set to a small epsilon value, $\varepsilon$, at any point belonging to the vessel tree and to a large value, say 20 times epsilon, at all the points that do not belong to the vessel tree. For a reference starting point $P_{ref}$ and any point $P_d$, the minimal path $C^*(P_{ref}, P_d)$ will link $P_{ref}$ to $P_d$ while achieving the smallest possible sum of all the potential values encountered on that path. In Fig. 10, a vessel tree 150 is illustrated, together with a reference point $P_{ref}$, a destination point $P_d$, and an optimal destination point $P^*_d$ A potential value equal to $\varepsilon$ is set at every point of the vessel tree and a potential value equal to $20 \times \varepsilon$ is set at every point that do not belong to the vessel tree. To every path $C(P_{ref}, P_d)$ linking $P_{ref}$ and $P_d$ corresponds a number $A(C(P_{ref}, P_d))$ equal to the sum of the potential values encountered on that path. The minimal path $C^*(P_{ref}, P_d)$ corresponds to the smallest possible value of A among all the possible paths from $P_{ref}$ to $P_d$. As mentioned above, this minimal path can be found efficiently using the Fast-Marching algorithm. Now, $d_T$ may be provided by the length of the minimal path $C^*(P_{ref}, P^*_d)$, where $P^*_d$ is the point belonging to the tree that maximizes $A(C^*(P_{ref}, P_d))$ over all the tree points $P_d$. In other words, $d_T$ corresponds to the longest minimal path, which is, from a geodesic distance point of view, the longest of the shortest paths starting from $P_{ref}$ and ending at a point of the tree. By way of an example, this length is illustrated as the length of the dashed line between the points A and B for the vessel 110 in Fig. 8. Point A corresponds to the position of the ostium in the vessel of interest, and point B corresponds to the most distal identifiable point in the vessel in the temporal sequence of angiographic images 130. The length of this vessel may be determined using any of the X-ray imaging system calibration techniques described above to provide a scaling between angiographic image pixels and a real-world dimension, such as millimetres or centimetres per pixel in the patient. Using the length of the longest vessel in the vessel tree as the corresponding transit length $d_T$ in this manner has been shown to provide a reliable estimate of the transit velocity, defined as the ratio of the transit length $d_T$ to the transit time $T_T$. This is because it is robust to changes in the vascular tree topology and does not overestimate the velocity in the case the contrast agent progresses simultaneously in two parallel branches.

The inventors have also determined that microcirculatory resistance values, such as the IMR, and which rely on the value of the transit time, may also be subject to errors from other sources. One further error source relates to the fact that the blood velocity varies throughout the cardiac cycle. A consequence of this is that measurements of the transit time $T_T$ are dependent on the portion of the cardiac cycle during which the transit time is measured. Yet another error source relates to the measurement of the transit length $d_T$ that is used to calculate the normalised microcirculatory resistance values described above. Further improvements to calculated microcirculatory resistance values may be achieved by addressing one or both of these error sources, as described in more detail below.

As mentioned above, the inventors have determined that measurements of the transit time are dependent on the portion of the cardiac cycle during which the transit time is measured. This is due to the fact that the blood velocity varies throughout the cardiac cycle. Consequently, measurements of the transit time $T_T$ during a portion of the systole phase differ from measurements of the transit time during a portion of the diastole phase, for example. This error may be compensated-for to some extent by repeating the measurement of the transit time multiple times, and averaging the transit times. However, the resulting complications to workflow associated with repeatedly injecting a bolus into the vessel and averaging the transit times are highly undesirable. Depending on the type of the bolus, the existing workflow complications may include: performing multiple injections of the bolus, performing multiple angiographic acquisitions with their associated increase in X-ray dose and increase contrast-agent, the difficulty in calculating such an average during a medical procedure, and the increased time of the medical procedure. Consequently it would be advantageous to make an accurate transit time measurement that obviates one or more of these issues.

In one example, the normalised microcirculatory resistance value is provided based on a corrected transit time $T'_T$. In this example, the injected bolus passes the proximal position $Pos_a$ in the vessel 110 at a proximal time $T_0$, and the injected bolus passes the distal position $Pos_d$ in the vessel 110 at a distal time $T_1$, and the injected bolus has an average transit velocity $V_T$ defined as a ratio of the transit length $d_T$ to the transit time $T_T$. In this example, the one or more processors 120 are further configured to correct the transit time $T_T$ such that the normalised microcirculatory resistance value is provided based on a corrected transit time $T'_T$. The one or more processors 120 are configured to provide the corrected transit time $T'_T$ by:

- receiving measured cardiac cycle data $C_m(t)$ for a heart fluidically coupled to the vessel 110, the measured cardiac cycle data $C_m(t)$ comprising a measured cardiac period $T_{Cm}$, and a time of a signature of each of one or more cardiac states SS, SD within the cardiac cycle;
- mapping the time of the one or more signatures, and the proximal time $T_0$, and the distal time $T_1$, to corresponding times within the measured cardiac period $T_{Cm}$;
- receiving reference fluid velocity data comprising a time-dependent reference fluid velocity curve $V_{ref}(t)$ representing a fluid velocity in the vessel 110 over a reference cardiac cycle having a reference cardiac period $T_{Cref}$;
- identifying in the reference fluid velocity curve $V_{ref}(t)$, a time of a reference signature corresponding to each of the one or more cardiac states SS, SD in the measured cardiac cycle data $C_m(t)$;
- transforming a time axis of the reference fluid velocity curve $V_{ref}(t)$ such that the reference cardiac period $T_{Cref}$ matches the measured cardiac period $T_{Cm}$, and such that the time of the reference signature of each of the one or more cardiac states SS, SD identified in the reference fluid velocity curve $V_{ref}(t)$ corresponds to the time of the signature of each of the one or more corresponding cardiac states SS, SD in the measured cardiac cycle data $C_m(t)$;
- transforming an amplitude axis of the transformed reference fluid velocity curve $V'_{ref}(t)$ to provide an amplitude-transformed reference fluid velocity curve $V''_{ref}(t)$ such that an average velocity $V_{Pref}$ of the amplitude-transformed reference fluid velocity curve $V''_{ref}(t)$ computed over a time interval between the proximal time $T_0$ and the distal time $T_1$, corresponds to the average transit velocity $V_T$; and
- providing a corrected transit time $T'_T$ by multiplying the transit time $T_T$ by a ratio of the average transit velocity $V_T$ to the average velocity $V_{refFull}$ of the amplitude-transformed reference fluid velocity curve $V''_{ref}(t)$ over a full cardiac cycle.

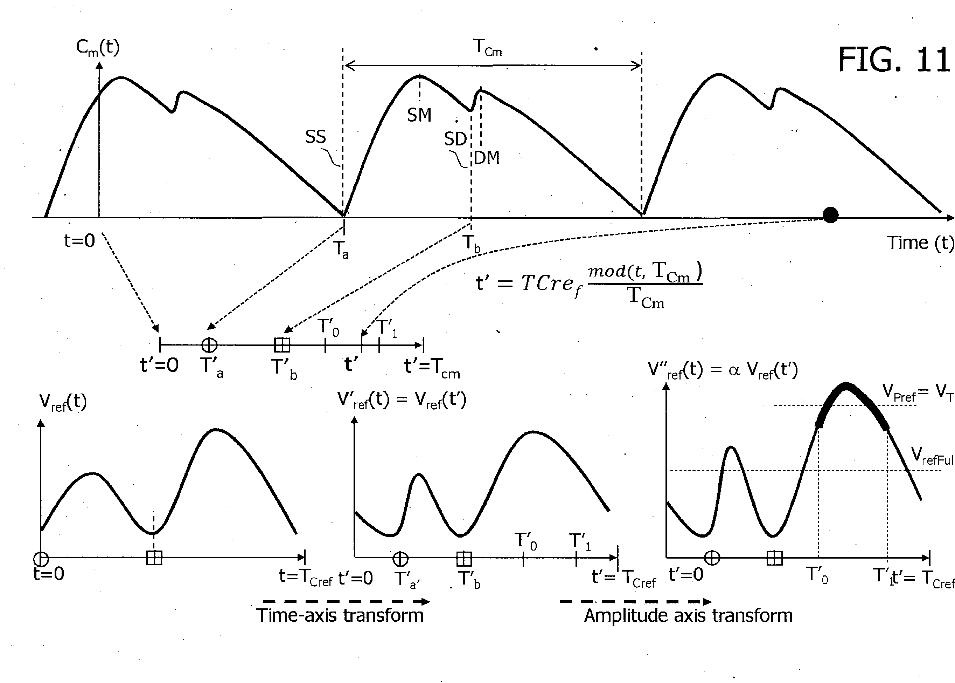

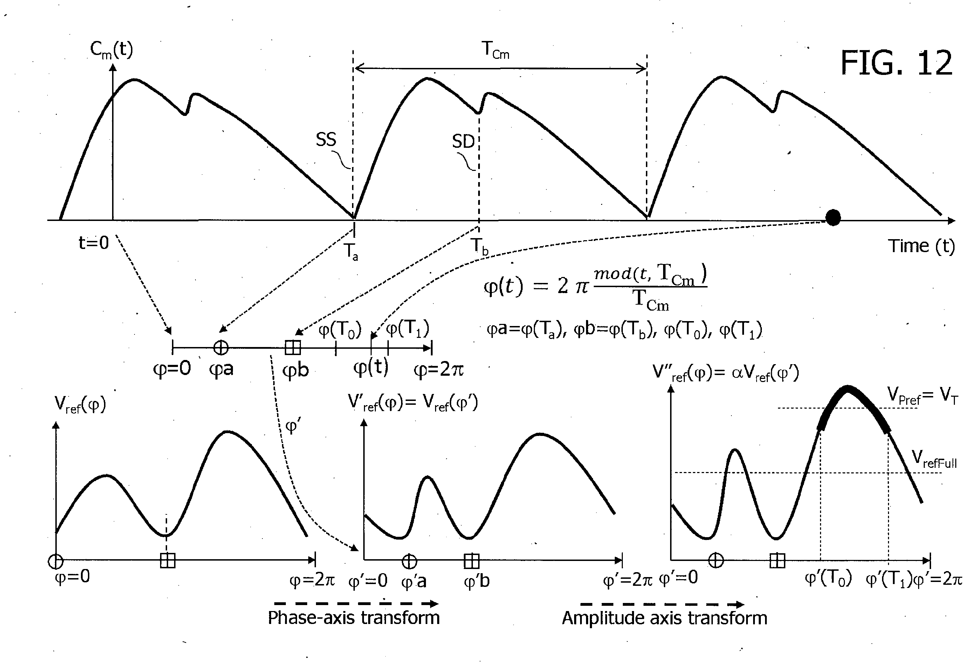

The operations performed in this example are described with reference to Fig. 11, which is a schematic diagram illustrating operations relating to a first example of correcting a transit time of a vessel, in accordance with some aspects of the present disclosure. The operations performed in this example may also be performed in an equivalent manner by the operations described below with reference to Fig. 12, and which are described in more detail below. It is noted that the use of the corrected transit time $T'_T$ described in relation to Fig. 11, is not limited to determining a normalised microcirculatory resistance value for a vessel. The corrected transit time $T'_T$ also finds application as a vessel parameter in the medical field in general.

With reference to the operation of receiving measured cardiac cycle data $C_m(t)$ in this example, the upper portion of Fig. 11 illustrates an example of measured cardiac cycle data $C_m(t)$. The cardiac cycle data $C_m(t)$ may be provided by various sources, including sensor data representing a pressure, or a velocity, or a temperature, of a fluid in the vessel 110; electrocardiogram data generated by one or more sensors in communication with the heart; and X-ray angiographic image data comprising a temporal sequence of images 130 representing the vessel 110. Various sensors are known for this purpose. Alternatively, X-ray angiographic image data may be analysed to provide the cardiac cycle data $C_m(t)$. For example, if the X-ray angiographic image data corresponds to a portion of the heart, a signal $C_m(t)$ may be determined from temporal changes in the intensity of angiographic images within the region representing the heart. The position of the coronary arteries may for example be detected in such images, and the intensity variations representing their movement may be detected in order to provide a signal that varies over time and which represents the heart cycle. In the example illustrated in Fig. 11, the cardiac cycle data $C_m(t)$ represents an intraluminal pressure.

The measured cardiac cycle data $C_m(t)$ may be received via any form of data communication, including wired, optical, and wireless communication. By way of some examples, when wired or optical communication is used, the communication may take place via signals transmitted on an electrical or optical cable, and when wireless communication is used, the communication may for example be via RF or optical signals.

The measured cardiac cycle data $C_m(t)$ illustrated in Fig. 11 illustrates a signal that varies continuously over time. The measured cardiac cycle data $C_m(t)$ also includes a measured cardiac period $T_{Cm}$, and a time of a signature of each of one or more cardiac states SS, SD within the cardiac cycle. A cardiac state is defined herein as a biological state of the heart. In the illustrated example, the intraluminal pressure signal has a period $T_{cm}$, and a signature of the start of the systolic phase, SS, and a signature of the start of the diastolic phase, SD. In general, the measured cardiac cycle data $C_m(t)$ may include a signature of one or more of such cardiac states. Depending on the origin of the measured cardiac cycle data $C_m(t)$, the measured cardiac cycle data $C_m(t$ may alternatively or additionally include a signature of one or more of the following cardiac states: an instant of maximum velocity DM during the diastole cardiac phase; and an instant of maximum velocity SM during the systole cardiac phase.

It is noted that whilst the measured cardiac cycle data $C_m(t)$ illustrated in Fig. 11 includes a signal that varies continuously over time, the measured cardiac cycle data $C_m(t)$ that is received by the one or more processors 120 might only include the measured cardiac period $T_{Cm}$, and a time of a signature of each of one or more cardiac states SS, SD within the cardiac cycle.

The operation of mapping the time of the one or more signatures, and the proximal time $T_0$, and the distal time $T_1$, to corresponding times within the measured cardiac period $T_{Cm}$, is illustrated in the central portion of Fig. 11. The equation in the central portion of Fig. 11 may be used to perform this mapping of the respective times to corresponding times within the measured cardiac period $T_{Cm}$. The proximal time $T_0$, and the distal time $T_1$ can be mapped to corresponding times $T_0'$ and $T_1'$ within the measured cardiac period $T_{Cm}$ by synchronising the measurement of these times to the time measurement in the measured cardiac cycle data $C_m(t)$. In the illustrated example, the proximal time $T_0$, and the distal time $T_1$ both occur in the diastole portion of the cardiac cycle.

An example of a time-dependent reference fluid velocity curve $V_{ref}(t)$ that is received in the operation of: receiving reference fluid velocity data comprising a time-dependent reference fluid velocity curve $V_{ref}(t)$ representing a fluid velocity in the vessel 110 over a reference cardiac cycle having a reference cardiac period $T_{Cref}$, is illustrated in the lower left-hand portion of Fig. 11. The reference fluid velocity curve $V_{ref}(t)$ is a model of the blood velocity, and may be obtained from literature, or it may be determined from clinical data. The reference fluid velocity curve $V_{ref}(t)$ may represent a vessel under specified conditions, wherein reference fluid velocity curve $V_{ref}(t)$ is selected to correspond to the vessel 110. For instance, the fluid velocity curve $V_{ref}(t)$ may be specified for a vessel, and for each of the basal state and the hyperemic state, and for a vessel patency. This accounts for factors such as the right coronary artery generally having a markedly less pronounced diastole flow component than the left coronary artery. The vessel patency may be defined by a Fractional Flow Reserve value. As illustrated in the lower left-hand portion of Fig. 11, the time-dependent reference fluid velocity curve $V_{ref}(t)$ has a period $T_{Cref}$.

The operation of identifying in the reference fluid velocity curve $V_{ref}(t)$, a time of a reference signature corresponding to each of the one or more cardiac states SS, SD in the measured cardiac cycle data $C_m(t)$; is also illustrated in the lower left-hand portion of Fig. 11. In the illustrated example, the time of a signature corresponding to the start of the systole phase, SS, is identified in the reference fluid velocity curve $V_{ref}(t)$ by means of a circle symbol. Likewise, the time of a signature corresponding to the start of the diastole phase, SD, is identified in the reference fluid velocity curve $V_{ret}(t)$ by means of a square symbol. The fact that these signatures in the reference fluid velocity curve $V_{ref}(t)$ represent these cardiac states, may be determined from clinical studies.

The operation of transforming a time axis of the reference fluid velocity curve $V_{ref}(t)$, is illustrated in the lower central portion of Fig. 11. The result, illustrated in the lower central portion of Fig. 11, is that the reference fluid velocity curve $V_{ret}(t)$ from the lower left-hand portion of Fig. 11, is mapped onto the time axis illustrated in the central portion of Fig. 11. This provides the transformed reference fluid velocity curve $V'_{ref}(t)$. This operation may include performing a linear mapping of the time axis of the reference fluid velocity curve $V_{ref}(t)$. In this case, the operation of transforming a time axis of the reference fluid velocity curve $V_{ref}(t)$, can be described by the equations:

$$t'(t) = \left(\frac{T_{Cref}}{T_{Cm}} \cdot mod(t, T_{Cm})\right).$$

Equation 5

$$V'_{ref}(t) = V_{ref}(t')$$

Equation 6

wherein mod(A,B) stands for A modulo B, i.e. A - E(A/B), with E(x) designating the integral part of x.

This provides a linear remapping of the temporal axis of the reference fluid velocity curve such that the period $T_{Cref}$ of the reference fluid velocity curve, matches the measured cardiac period $T_{Cm}$ of the measured cardiac cycle data $C_m(t)$.

Alternatively, this operation may include performing separate piecewise linear mapping of the time axis of the reference fluid velocity curve $V_{ref}(t)$ for each time interval between the signatures of consecutive selected cardiac states. By way of an example, if the two time intervals either side of the start of the diastole phase, SD, are mapped in this manner, the operation of transforming a time axis of the reference fluid velocity curve $V_{ref}(t)$, can be described by the equations:

$$t \leq SD: t'(t) = \left(\frac{SD_{ref}}{SD} \cdot t\right)$$

Equation 7

$$t > SD: t'(t) = \left(\frac{(T_{Cref} - SD_{ref})}{(T_{Cm} - SD)} \cdot (t - SD) + SD_{ref}\right)$$

Equation 8

together with Equation 6 defined above.

In Equation 7 and Equation 8, SD and $SD_{ref}$ are the times of the start of the diastole phase in the measured cardiac cycle data $C_m(t)$, and in the reference fluid velocity curve, respectively.

The operation of transforming an amplitude axis of the transformed reference fluid velocity curve $V'_{ref}(t)$ to provide an amplitude-transformed reference fluid velocity curve $V''_{ref}(t)$, is illustrated in the lower right-hand portion of Fig. 11. This operation may include linearly scaling the amplitude axis of the transformed reference fluid velocity curve V'ret(t) for instance according to the equation:

$$V''_{ref}(t) = \alpha V'_{ref}(t) = \alpha V_{ref}(t')$$

Equation 9

where $\alpha$ is the amplitude scaling factor.

This operation may be performed iteratively until the average velocity $V_{Pref}$ of the amplitude-transformed reference fluid velocity curve $V''_{ref}(t)$ computed over a time interval between the proximal time $T_0$ and the distal time $T_1$, corresponds to the average transit velocity $V_T$.

A corrected transit time $T'_T$ is then provided by multiplying the transit time $T_T$ by a ratio of the average transit velocity $V_T$ to the average velocity $V_{refFull}$ of the amplitude-transformed reference fluid velocity curve $V''_{ref}(t)$ over a full cardiac cycle.

The result of these operations is to provide a corrected transit time $T'_T$ that is independent of the portion of the cardiac cycle during which the transit time $T_T$ is measured. Thus, it obviates some of the existing workflow complications associated with repeatedly injecting a bolus into the vessel and averaging the transit times.

As mentioned above, the inventors have determined that another error source relates to the measurement of the transit length $d_T$ that is used to calculate the normalised microcirculatory resistance values described above. In one example, the normalised microcirculatory resistance value is provided by using an estimate of the transit length $d_T$ that is determined by analysing X-ray angiographic image data. In this example, the one or more processors 120 are configured to receive X-ray angiographic image data comprising a temporal sequence of images 130 representing a flow of the injected bolus through the vessel 110; and the one or more processors 120 are configured to analyse the X-ray image data to determine the transit time $T_T$ and an estimate of the transit length $d_T$. The images 130 comprise projection images generated by an X-ray imaging system; and the one or more processors 120 are further configured to:

- receive X-ray imaging system geometric data representing an orientation of the X-ray imaging system respective the vessel 110; and
- determine the estimate of the transit length $d_T$ by:
- determining a length scale factor for positions along a length of the vessel 110 in one or more of the X-ray projection images, based on a matching between the vessel 110 in the one or more of the X-ray projection images, and a reference vessel in a reference projection image generated by projecting a 3D model of a reference vessel 170 representing the vessel 110 using the X-ray imaging system geometric data; and
- computing the length of the vessel 110 between the proximal position $Pos_a$ and the distal position $Pos_d$ by scaling the vessel 110 represented in the one or more X-ray projection images, with the length scale factor determined at the corresponding positions along the length of the vessel 110, to provide the estimate of the transit length $d_T$.

In so doing, an accurate transit length $d_T$, is provided. The transit length $d_T$ is accurate because it takes account of foreshortening. By using the transit length that is calculated in this manner, in order to compute the above-described normalised microcirculatory resistance values, the accuracy of these values may be improved. It is noted that the use of the length scale factor that is determined in this manner, is not limited to computing a normalised microcirculatory resistance value for a vessel. The length scale factor may also be used to compute a length of a portion of a vessel in the medical field in general.

This example is described with reference to Fig. 2 and Fig. 13 - Fig. 16. With reference to Fig. 2, the X-ray angiographic image data may be received from the X-ray imaging system 220. The injected bolus in this example is a contrast agent bolus. The X-ray angiographic image data may be received via any form of data communication, including wired, optical, and wireless communication. By way of some examples, when wired or optical communication is used, the communication may take place via signals transmitted on an electrical or optical cable, and when wireless communication is used, the communication may for example be via RF or optical signals.

By way of some examples, the received X-ray imaging system geometric data may include one or more of:

- a rotational angle $\alpha$ of a central ray of the X-ray imaging system around a longitudinal axis of a subject; and/or
- a tilt angle $\beta$ of the central ray of the X-ray imaging system with respect to a cranial-caudal axis of the subject; and/or
- a pixel size of the X-ray detector 220b of the X-ray imaging system 220; and/or
- a length or a width of a detector 220b of the X-ray imaging system, and a separation between an X-ray source 220a and the X-ray detector 220b of the X-ray imaging system 220, and a separation between the X-ray source 220a and the vessel 110.

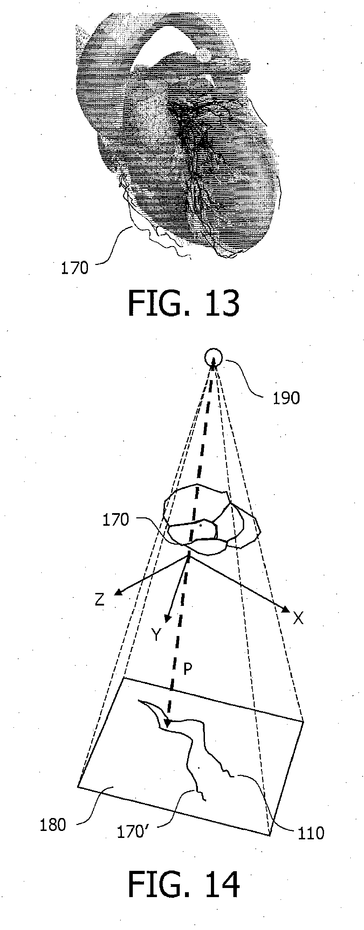

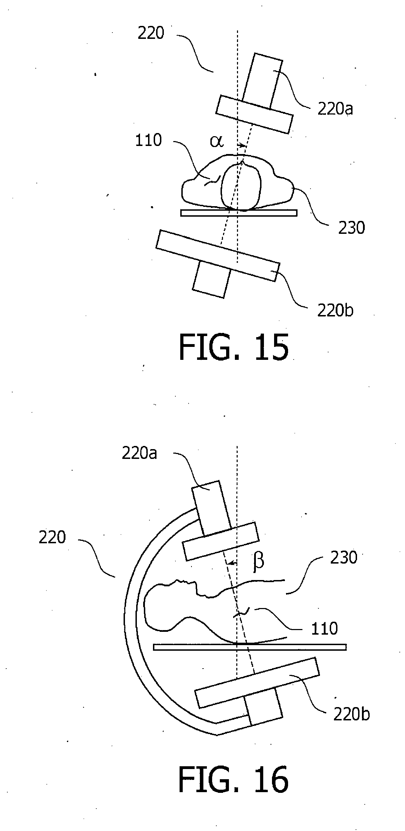

Examples of a rotational angle $\alpha$, and a tilt angle $\beta$, are illustrated respectively in Fig 14 and Fig..16. Fig. 15 is a schematic diagram illustrating X-ray imaging system geometric data representing an orientation of an X-ray imaging system 220 respective a portion of a vessel 110, including a rotational angle $\alpha$ of a central ray of the X-ray imaging system 220 around a longitudinal axis of a subject 230, in accordance with some aspects of the present disclosure. Fig. 16 is a schematic diagram illustrating X-ray imaging system geometric data representing an orientation of an X-ray imaging system 220 respective the portion of the vessel 110, including a tilt angle $\beta$ of a central ray of the X-ray imaging system 220 with respect to a cranial-caudal axis of the subject 230, in accordance with some aspects of the present disclosure.

In Fig. 15 and Fig. 16, the illustrated vessel 110 forms part of a subject 230, and the X-ray imaging system 220 includes an X-ray source 220a and an X-ray detector 220b.

In this example, the operation of determining a length scale factor for positions along a length of the vessel 110 in one or more of the X-ray projection images, is illustrated with reference to Fig. 13 and Fig. 14. In some examples, the length scale factor for positions along a length of the vessel 110 is determined in one and only one of the X-ray projection images. The operation of determining a length scale factor includes projecting a 3D model of a reference vessel 170 representing the vessel 110 using the X-ray imaging system geometric data. Fig. 13 is a schematic diagram illustrating a 3D model of a reference vessel 170, in accordance with some aspects of the present disclosure. The 3D model illustrated in Fig. 13 includes multiple heart vessels. The labelled vessel 110 represents the right coronary artery, RCA. Fig. 14 is a schematic diagram illustrating a projected 3D model of a reference vessel 170' on a virtual plane 180 of a detector of an X-ray imaging system, from a perspective of an X-ray source 190 of the X-ray imaging system, in accordance with some aspects of the present disclosure. The projected 3D model of a reference vessel 170' is generated using the X-ray imaging system geometric data. The projected 3D model of the reference vessel 170' may be generated using data such as the rotational angle $\alpha$, the tilt angle $\beta$, the length or the width of a detector 220b of the X-ray imaging system, the separation between the X-ray source 220a and the X-ray detector 220b of the X-ray imaging system 220, and the separation between the X-ray source 220a and the vessel 110. The projected 3D model provides an accurate depiction of the reference vessel 170, on the virtual plane 180 of the detector 220b, as it would appear on the detector of the X-ray imaging system.

The operation of determining a length scale factor for positions along a length of the vessel 110 includes matching the vessel 110 in the one or more of the X-ray projection images, and the projected reference vessel 170'. The vessel 110 in the projection image is also illustrated as an overlay on the virtual plane 180 of the detector 220b in Fig. 14. In the illustrated example, the matching operation has not been completed, and thus there is a discrepancy between the the projected reference vessel 170' and the vessel 110. In Fig. 14, the discrepancy should therefore be reduced in order to obtain a match. This may involve aligning the 3D model so that the ostium or starting point of the reference vessel corresponds, after projection, to the ostium or starting point of the vessel 110. This may involve a scaling of the 3D model so that anatomical features of the model, after projection, correspond to the same anatomical features in the vessel image. This may involve selecting a different 3D model or part of the 3D model of the reference vessel, and projecting this model or part of this model in a similar manner, until an improved match is found 170 representing the vessel 110.

Having found a closest match, a length scale factor for positions along a length of the vessel 11.0, is determined. The length scale factor may be determined from the 3D model of the reference vessel 170 since the length of the vessel in this model is known.

The length of the vessel 110 between the proximal position $Pos_a$ and the distal position $Pos_d$ is then computed by scaling the vessel 110 represented in the one or more X-ray projection images, with the length scale factor determined at the corresponding positions along the length of the vessel 110, to provide the estimate of the transit length $d_T$.

The operations of determining a length scale factor for positions along a length of the vessel 110, and computing the length of the vessel 110 between the proximal position $Pos_a$ and the distal position $Pos_d$, are described in more detail in the example implementation below.

In this implementation, three-dimensional centerlines of vessels, for example the coronary artery trees, of multiple - different subjects are stored as sequences of 3D coordinates. The centerlines can be determined using known imaging and image processing methods, such as for example using cardiac CT angiography images and Philips IntelliSpace Portal Comprehensive Cardiac Analysis software. Vessel segments may be labelled using a standardized naming scheme, such as for example the AHA 15 segment model-for coronary arteries disclosed in a document by Austen, W. G., et al., entitled "A reporting system on patients evaluated for coronary artery disease", Report of the Ad Hoc Committee for Grading of Coronary Artery Disease, Council on Cardiovascular Surgery, American Heart Association. (1975) Circulation. 51 (4 Suppl): 5-40. The labelling may be performed by assigning a unique label to each centerline point of the coronary tree. An expert user may perform this operation. Vessel branching points are identified as locations where the labelling changes along the tree. The database can be built as a set of individual trees, or as a single model of the average tree, or as average models per tree type, e.g. average left dominant model, right dominant model, etc.

Since vessel segment centerlines are represented as sequences of 3D coordinates $P_{i,s}$, $(i = 0..N)$, the 3D length $l_{3D}(s)$ of a vessel segment s can be calculated as the sum of the Euclidean distances $d(P_i, P_{i-1})$ between adjacent centerline points:

$$l_{3D}(s) = \sum_{i=1}^{N} d\left(P_{i,s}, P_{i-1,s}\right) \qquad \text{Equation 10}$$

In addition to being vessel-specific, the 3D vessel segments might be further specified by other criteria such as the patient's gender. When used to predict foreshortening for an observed 2D centreline, those additional criteria can be used to select the correct database model.

In projection images, vessel segments are extracted by known methods. In the two-dimensional case, vessel segments s are represented as sequences of 2D detector coordinates $X_i$, $(i = 0..M)$. The observed 2D length of a vessel segment $l_{2D}(s)$, including foreshortening and magnification by the X-ray fan beam, can then be calculated as the sum of the Euclidean distances $d(X_i, X_{i-1})$ between adjacent 2D centerline points, multiplied with a calibration factor $d_{cal}$ that estimates the pixel-to-millimeter correspondence for a given object and projection geometry:

$$l_{2D}(s) = \sum_{i=1}^{M} d(X, X_{i-1}) \cdot d_{cal} \qquad \text{Equation 11}$$

The 2D vessel segments use the same vessel segment labelling as the database of 3D vessel segments. The calibration factor $d_{cal}$ can be estimated from the known size of an injection catheter in the angiogram or other known calibration methods.

As illustrated in Fig. 15 and Fig. 16, X-ray angiographic images are typically generated using an X-ray imaging system that includes a C-arm and a fan beam. Therefore, the projection unit can be defined by the location of the X-ray focal spot, the rotation iso-center of the C-arm, the corner location of the detector as well as its size and number of pixels in each direction. Of particular interest for foreshortening are the rotation angle $\alpha$ of the central ray around the patient's long axis (RAO-LAO direction), and the tilt angle $\beta$ in the cranial-caudal direction. The two angles determine the 3D location of the focal spot $F(\alpha,\beta)$ for a projection using a given system geometry.

The foreshortening estimation starts by selecting the appropriate 3D vessel centerline model as determined by the segment label *s*. First, the $P_{i,s}$ of the chosen centerline model are shifted by an offset to ensure that $P_{0,s}$ is on the line connecting $F(\alpha,\beta)$ and $X_0$, and that the center of mass of the segment is in a plane through the iso-center and parallel to the detector of the C-arm system (depth estimation). The shifted centerline is then $P_{i,s}^{X_0}$, (*i* = 0..*N*).

The true 3D length $l_{3D}(s)$ of the segment model is known. The foreshortened and projected length $l_{3D}^{p}(s;\alpha,\beta,X_0)$ of the segment can be calculated using simple ray geometry to project the $P_{i,s}^{X_0}$ onto the detector plane, resulting in the projected points $\prod\left(P_{i,s}^{X_0};\alpha,\beta\right)$ such that:

$$l_{3D}^{p}(s;\alpha,\beta,X_0) = \sum_{i=1}^{N} d\left(\prod\left(P_{i,s}^{X_0};\alpha,\beta\right), \prod\left(P_{i-1,s}^{X_0};\alpha,\beta\right)\right) \quad \text{Equation 12}$$

Using the ratio of the true 3D length and foreshortened and projected length, the vessel segment length $l_{2D}(s)$ observed in 2D can be compensated for foreshortening and magnification as follows:

$$l_{2D}^{comp}(s;\alpha,\beta,X_0) = l_{2D}(s) \cdot \frac{l_{3D}^{p}(s;\alpha,\beta,X_0)}{l_{3D}(s)} \quad \text{Equation 13}$$

This compensated length can then be used when the true 3D length of the vessel is required for the most accurate results.

It is noted that the system 100 described above may also include one or more of: an injector 210, X-ray imaging system 220, a patient bed 240, a display 250, and on which one or more of the computed values may be displayed, and a user input device (not illustrated). The user input device may be a keyboard, a mouse, a touchscreen, and so forth.

In another example, a method of providing a normalised microcirculatory resistance value for a vessel 110, is provided. The method comprises:

- computing S110 a microcirculatory resistance value for the vessel 110 based on a transit time $T_T$ taken for an injected bolus to travel between a proximal position $Pos_a$ in the vessel, and a distal position $Pos_d$ in the vessel; and
- dividing S120 the computed microcirculatory resistance value by a transit length $d_T$ representing a length of the vessel between the proximal position $Pos_a$ and the distal position $Pos_d$, to provide the normalised microcirculatory resistance value.

As previously mentioned, the use of the corrected transit time $T'_T$ described in relation to Fig. 11, is not limited to determining a normalised microcirculatory resistance value for a vessel. The corrected transit time $T'_T$ also finds application as a vessel parameter in the medical field in general.

Thus, in accordance with a second aspect of the present disclosure, a system for correcting a transit time $T_T$ of a vessel 110, is provided. Various examples, in accordance with this second aspect of the present disclosure, are enumerated as Example 1A - Example 15A below.

Example 1A. A system for correcting a transit time $T_T$ of a vessel 110, the transit time $T_T$ representing a time taken for an injected bolus to travel along a transit length $d_T$ of the vessel 110 between a proximal position $Pos_a$ in the vessel at a proximal time $T_0$, and a distal position $Pos_d$ in the vessel at a distal time $T_1$, and at an average transit velocity $V_T$ defined as a ratio of the transit length $d_T$ to the transit time $T_T$, the system comprising one or more processors configured to:

- receive S210 measured cardiac cycle data $C_m(t)$ for a heart fluidically coupled to the vessel 110, the measured cardiac cycle data $C_m(t)$ comprising a measured cardiac period $T_{Cm}$, and a time $T_a$, $T_b$ of a signature of each of one or more cardiac states SS, SD within the cardiac cycle;
- map S220 the time of the one or more signatures, and the proximal time $T_0$, and the distal time $T_1$, to corresponding times within the measured cardiac period $T_{Cm}$;
- receive S230 reference fluid velocity data comprising a time-dependent reference fluid velocity curve $V_{ref}(t)$ representing a fluid velocity in the vessel 110 over a reference cardiac cycle having a reference cardiac period $T_{Cref}$;

- identify S240 in the reference fluid velocity curve $V_{ref}(t)$, a time of a reference signature corresponding to each of the one or more cardiac states SS, SD in the measured cardiac cycle data $C_m(t)$;
- transform S250 a time axis of the reference fluid velocity curve $V_{ref}(t)$ such that the reference cardiac period $T_{Cref}$ matches the measured cardiac period $T_{Cm}$, and such that the time of the reference signature of each of the one or more cardiac states SS, SD identified in the reference fluid velocity curve $V_{ref}(t)$ corresponds to the time of the signature of each of the one or more corresponding cardiac states SS, SD in the measured cardiac cycle data $C_m(t)$;
- transform S260 an amplitude axis of the transformed reference fluid velocity curve $V'_{ref}(t)$ to provide an amplitude-transformed reference fluid velocity curve $V''_{ref}(t)$ such that an average velocity $V_{Pref}$ of the amplitude-transformed reference fluid velocity curve $V''_{ref}(t)$ computed over a time interval between the proximal time $T_0$ and the distal time $T_1$, corresponds to the average transit velocity $V_T$; and
- provide S270 a corrected transit time $T'_T$ by multiplying the transit time $T_T$ by a ratio of the average transit velocity $V_T$ to the average velocity $V_{refFull}$ of the amplitude-transformed reference fluid velocity curve $V''_{ref}(t)$ over a full cardiac cycle.

Example 2A. The system according to Example 1A, wherein the measured cardiac cycle data $C_m(t)$ comprises a time of a signature of each of two or more cardiac states selected from: a start of a systole cardiac phase SS, a start of a diastole cardiac phase SD, an instant of maximum velocity DM during the diastole cardiac phase, and an instant of maximum velocity during the systole cardiac phase SM, and wherein the one or more processors are further configured to:

- transform the time axis of the reference fluid velocity curve $V_{ref}(t)$ such that the times of the reference signatures identified in the reference fluid velocity curve $V_{ref}(t)$ correspond to the times of the corresponding signatures in the measured cardiac cycle data $C_m(t)$.

Example 3A. The system according to Example 1A, wherein the one or more processors are configured to transform the time axis of the reference fluid velocity curve $V_{ref}(t)$ by performing a separate piecewise linear mapping of the time axis of the reference fluid velocity curve $V_{ref}(t)$ for each time interval between the signatures of consecutive selected cardiac states.

Example 4A. The system according to any one of Example 1A - Example 3A, wherein the one or more processors are further configured to identify the reference fluid velocity data by selecting the time-dependent reference fluid velocity curve $V_{ref}(t)$ from a database of time-dependent reference fluid velocity curves for the vessel 110.

Example 5A. The system according to Example 4A, wherein the vessel 110 forms part of a patient, and wherein the selecting the time-dependent reference fluid velocity curve Vret(t) from a database of time-dependent reference fluid velocity curves for the vessel 110, is based on at least one of: a type of the vessel, a dimension of the vessel, a body mass index of the patient, a gender of the patient, an age of the patient, a type of vessel dominance in the patient, an hemodynamic state of the patient, a calcification index of the patient, a plaque burden index of the patient, a vessel stiffness parameter of the patient, a cardiac function parameter of the patient.

Example 6A. The system according to any one of Example 1A - Example 5A, wherein the measured cardiac cycle data $C_m(t)$ comprises at least one of:

- X-ray angiographic image data comprising a temporal sequence of images 130 representing the vessel 110;
- sensor data representing a pressure, or a velocity, or a temperature, of a fluid in the vessel 110; and
- electrocardiogram data generated by one or more sensors in communication with the heart; and
- wherein the one or more processors are configured to determine the measured cardiac period $T_{Cm}$, and the time of the signature of each of the one or more cardiac states SS, SD within the cardiac cycle, by analysing the X-ray angiographic image data, the sensor data, and the electrocardiogram data, respectively.

Example 7A. The system according to any one of Example 1A - Example 6A, wherein the one or more processors are further configured to compute a microcirculatory resistance value, for the vessel 110, the microcirculatory resistance value being based on the transit time $T_T$, and wherein the microcirculatory resistance value is computed using the corrected transit time $T'_T$.

Example 8A. The system according to Example 7A, wherein the microcirculatory resistance value is an index of microcirculatory resistance, IMR, value.

**Example 9A.** The system according to any one of Example 1A - Example 8A, wherein the one or more processors are further configured to:

- receive intraluminal device data, the intraluminal device data comprising proximal sensor data generated by a proximal sensor disposed at the proximal position $Pos_a$ in the vessel 110, and distal sensor data generated by a distal sensor disposed at the distal position $Pos_d$ in the vessel; and
- wherein the one or more processors are further configured to determine the transit time $T_T$ based on a time difference between a signal generated by the bolus at the proximal sensor and a signal generated by the bolus at the distal sensor.

**Example 10A.** The system according to any one of Example 1A - Example 9A, wherein the proximal sensor, and the distal sensor, comprise at least one of: a pressure sensor, a fluid velocity sensor, a temperature sensor, and an ultrasound sensor.

**Example 11A.** The system according to Example 9A or Example 10A, wherein the one or more processors are further configured to:

- receive X-ray image data, the X-ray image data comprising one or more images representing the proximal position $Pos_a$ of the proximal sensor and the distal position $Pos_d$ of the distal sensor; and
- analyse the one or more images to determine an estimate of the transit length $d_T$ based on a detection of the proximal position $Pos_a$ of the proximal sensor and the distal position $Pos_d$ of the distal sensor.

**Example 12A.** The system according to any one of Example 1A - Example 8A, wherein the injected bolus comprises an injected contrast agent bolus 140, and wherein the one or more processors are further configured to:

- receive X-ray angiographic image data comprising a temporal sequence of images 130 representing a flow of the injected bolus 140 through the vessel 110; and
- analyse the temporal sequence of images 130 to determine the transit time $T_T$ and/or an estimate of the transit length $d_T$.

**Example 13A.** The system according to Example 12A, wherein the one or more processors are further configured to identify the proximal position $Pos_a$ and the distal position $Pos_d$ in the vessel 110 in the X-ray angiographic image data; and

- determine the transit time $T_T$ based on the time taken for the injected contrast agent bolus 140 to travel between the identified proximal position $Pos_a$ in the vessel, and the identified distal position $Pos_d$ in the vessel, in the X-ray angiographic image data; and/or
- determine the estimate of the transit length $d_T$ based on a length of the vessel 110 between the proximal position $Pos_a$ and the distal position $Pos_d$, in the X-ray angiographic image data.

**Example 14A.** The system according to Example 13A, wherein the proximal position $Pos_a$ corresponds to a position of a detected front of the injected contrast agent bolus 140 in the vessel 110 in an earlier X-ray image in the temporal sequence 130, and wherein the distal position $Pos_d$ corresponds to a position of the detected front in a later X-ray image in the temporal sequence 130; and

- wherein the transit time $T_T$ is determined by computing a time difference between the earlier X-ray image and the later X-ray image; and
- wherein the estimate of the transit length $d_T$ is determined by:
- mapping the proximal position $Pos_a$ from the earlier image to the later image to provide a mapped proximal position $Pos'_a$ in the later image, or by mapping the distal position $Pos_d$ from the later image to the earlier image to provide a mapped distal position $Pos'_d$ in the earlier image; and
- calculating a length of the vessel 110 between the mapped proximal position $Pos'_a$ and the distal position $Pos_d$ in the later image, or between the proximal position $Pos_a$ and the mapped distal position $Pos'_d$ in the earlier image, respectively.

**Example 15A.** The system according to any one of Example 1A - Example 8A, wherein the one or more processors are further configured to receive the transit length $d_T$ and/or the transit time $T_T$ from a computer readable storage medium, or from an intraluminal sensing device, or from a user input device.

Thus, in accordance with the second aspect of the present disclosure, a system for correcting a transit time $T_T$ of a vessel 110, is provided. The transit time $T_T$ represents a time taken for an injected bolus to travel along a transit length $d_T$ of the vessel 110 between a proximal position $Pos_a$ in the vessel at a proximal time $T_0$, and a distal position $Pos_d$ in the vessel at a distal time $T_1$, and at an average transit velocity $V_T$ defined as a ratio of the transit length $d_T$ to the transit time $T_T$, the system comprising one or more processors configured to:

- receive S210 measured cardiac cycle data $C_m(t)$ for a heart fluidically coupled to the vessel 110, the measured cardiac cycle data $C_m(t)$ comprising a measured cardiac period $T_{Cm}$, and a time $T_a$, $T_b$ of a signature of each of one or more cardiac states SS, SD within the cardiac cycle;
- map S220 the time of the one or more signatures, and the proximal time $T_0$, and the distal time $T_1$, to corresponding times within the measured cardiac period $T_{Cm}$;
- receive S230 reference fluid velocity data comprising a time-dependent reference fluid velocity curve $V_{ref}(t)$ representing a fluid velocity in the vessel 110 over a reference cardiac cycle having a reference cardiac period $T_{Cref}$;
- identify S240 in the reference fluid velocity curve $V_{ref}(t)$, a time of a reference signature corresponding to each of the one or more cardiac states SS, SD in the measured cardiac cycle data $C_m(t)$;
- transform S250 a time axis of the reference fluid velocity curve $V_{ref}(t)$ such that the reference cardiac period $T_{Cref}$ matches the measured cardiac period $T_{Cm}$, and such that the time of the reference signature of each of the one or more cardiac states SS, SD identified in the reference fluid velocity curve $V_{ref}(t)$ corresponds to the time of the signature of each of the one or more corresponding cardiac states SS, SD in the measured cardiac cycle data $C_m(t)$;
- transform S260 an amplitude axis of the transformed reference fluid velocity curve $V'_{ref}(t)$ to provide an amplitude-transformed reference fluid velocity curve $V''_{ref}(t)$ such that an average velocity $V_{Pref}$ of the amplitude-transformed reference fluid velocity curve $V''_{ref}(t)$ computed over a time interval between the proximal time $T_0$ and the distal time $T_1$, corresponds to the average transit velocity $V_T$; and
- provide S270 a corrected transit time $T'_T$ by multiplying the transit time $T_T$ by a ratio of the average transit velocity $V_T$ to the average velocity $V_{refFull}$ of the amplitude-transformed reference fluid velocity curve $V''_{ref}(t)$ over a full cardiac cycle.

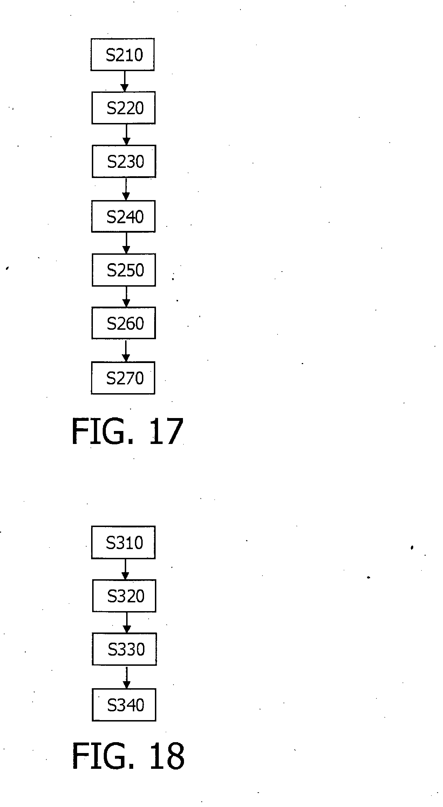

The result of these operations is to provide a corrected transit time $T'_T$ that is independent of the portion of the cardiac cycle during which the transit time $T_T$ is measured. Thus, it obviates some of the existing workflow complications associated with repeatedly injecting a bolus into the vessel and averaging the transit times. These operations correspond to the operations described above with reference to Fig. 11 and Fig. 2. These operations are also illustrated in Fig. 17, which is a flowchart illustrating an example of a method of correcting a transit time of a vessel, in accordance with some aspects of the present disclosure. Operations performed in accordance with the system for correcting a transit time $T_T$ of a vessel 110, may also be performed by the method illustrated in Fig. 17, and vice versa.

As described above, in Fig. 11, the corrected transit time $T'_T$ was provided, in-part, by the operation S220, wherein the time of the one or more signatures, and the proximal time $T_0$, and the distal time $T_1$, are mapped to corresponding times within the measured cardiac period $T_{Cm}$. A corrected transit time $T'_T$ may alternatively be provided in an equivalent manner by mapping the time of the one or more signatures, and the proximal time $T_0$, and the distal time $T_1$, to corresponding angular phases 0, $2\pi$ within the cardiac cycle. Mapping to corresponding angular phases, rather than to corresponding times, within the cardiac cycle has the same effect, the operations being performed in the phase domain rather than in the time domain.

With reference to Fig. 12, a system for correcting a transit time $T_T$ of a vessel, is provided. The transit time $T_T$ represents a time taken for an injected bolus to travel along a transit length $d_T$ of the vessel between a proximal position in the vessel at a proximal time $T_0$, and a distal position in the vessel at a distal time $T_1$, and at an average transit velocity $V_T$ defined by the ratio of the transit-length $d_T$ to the transit-time $T_T$. The system comprises one or more processors configured to:

- receive measured cardiac cycle data $C_m(t)$ for a heart fluidically coupled to the vessel, the measured cardiac cycle data $C_m(t)$ comprising a time $T_a$, $T_b$ of a signature of each of one or more cardiac states SS, SD within the cardiac cycle;
- map the time of the one or more signatures, and the proximal time $T_0$, and the distal time $T_1$, to corresponding angular phases 0, $2\pi$ within the cardiac cycle;
- receive reference fluid velocity data comprising an angular phase-dependent reference fluid velocity curve $V_{ref}(\varphi)$ representing a fluid velocity in the vessel over a reference cardiac cycle;
- identify in the reference fluid velocity curve $V_{ref}(\varphi)$, an angular phase of a reference signature corresponding to each of the one or more cardiac states SS, SD in the measured cardiac cycle data $C_m(t)$;
- transform a phase-axis of the reference fluid velocity curve $V_{ref}(\varphi)$ such that the angular phase of the reference signature of each of the one or more cardiac states SS, SD identified in the reference fluid velocity curve $V_{ref}(\varphi)$ corresponds to the angular phase of the signature of each of the one or more corresponding cardiac states SS, SD

in the measured cardiac cycle data $C_m(t)$;
- transform an amplitude axis of the transformed reference fluid velocity curve $V'_{ref}(\varphi)$ to provide an amplitude-transformed reference fluid velocity curve $V''_{ref}(\varphi)$ such that an average velocity $V_{Pref}$ of the amplitude-transformed reference fluid velocity curve $V''_{ref}(\varphi)$ computed over an angular phase interval starting at an angular phase corresponding to the proximal time $T_0$ and ending at an angular phase corresponding to the distal time $T_1$, corresponds to the average transit velocity $V_T$;
- provide a corrected transit-time $T'_T$ by multiplying the transit time $T_T$ by a ratio of the average transit velocity $V_T$ to the average velocity $V_{refFull}$ of the amplitude-transformed reference fluid velocity curve $V''_{ref}(\varphi)$ over a full cardiac cycle.

In so doing, a corrected transit time $T'_T$ that is independent of the portion of the cardiac cycle during which the transit time $T_T$ is measured. This obviates some of the existing workflow complications associated with repeatedly injecting a bolus into the vessel and averaging the transit times. These operations are described with reference to Fig. 12, which is a schematic diagram illustrating operations relating to a second example of correcting a transit time of a vessel, in accordance with some aspects of the present disclosure.

With reference to the operation of receiving measured cardiac cycle data $C_m(t)$ in this example, the upper portion of Fig. 12 illustrates an example of measured cardiac cycle data $C_m(t)$. The cardiac cycle data $C_m(t)$ may be provided by various sources, including sensor data representing a pressure, or a velocity, or a temperature, of a fluid in the vessel 110; electrocardiogram data generated by one or more sensors in communication with the heart; and X-ray angiographic image data comprising a temporal sequence of images 130 representing the vessel 110. Various sensors are known for this purpose. Alternatively, X-ray angiographic image data may be analysed to provide the cardiac cycle data $C_m(t)$. For example, if the X-ray angiographic image data corresponds to a portion of the heart, a signal $C_m(t)$ may be determined from temporal changes in the intensity of angiographic images within the region representing the heart. The position of the coronary arteries may for example be detected in such images, and the intensity variations representing their movement may be detected in order to provide a signal that varies over time and which represents the heart cycle. In the example illustrated in Fig. 12, the cardiac cycle data $C_m(t)$ represents an intraluminal pressure.

The measured cardiac cycle data $C_m(t)$ may be received via any form of data communication, including wired, optical, and wireless communication. By way of some examples, when wired or optical communication is used, the communication may take place via signals transmitted on an electrical or optical cable, and when wireless communication is used, the communication may for example be via RF or optical signals.

The measured cardiac cycle data $C_m(t)$ illustrated in Fig. 12 illustrates a signal that varies continuously over time. The measured cardiac cycle data $C_m(t)$ also includes a time $T_a$, $T_b$ of a signature of each of one or more cardiac states SS, SD within the cardiac cycle. A cardiac state is defined as a biological state of the heart. In the illustrated example, the intraluminal pressure signal has a period $T_{Cm}$, and a signature of the start of the systolic phase, SS, at time $T_a$, and a signature of the start of the diastolic phase, SD, at time $T_b$. In general, the measured cardiac cycle data $C_m(t)$ may include a signature of one or more of such cardiac states. Depending on the origin of the measured cardiac cycle data $C_m(t)$, the measured cardiac cycle data $C_m(t)$ may alternatively or additionally include a signature of one or more of the following cardiac states: an instant of maximum velocity DM during the diastole cardiac phase; and an instant of maximum velocity SM during the systole cardiac phase.

It is noted that whilst the measured cardiac cycle data $C_m(t)$ illustrated in Fig. 12 includes a signal that varies continuously over time, the measured cardiac cycle data $C_m(t)$ that is received by the processor might only include the time of the signature of each of one or more cardiac states SS; SD within the cardiac cycle.

The operation of mapping the time of the one or more signatures, and the proximal time $T_0$, and the distal time $T_1$, to corresponding angular phases 0, $2\pi$ within the cardiac cycle, is illustrated in the central portion of Fig. 12, in particular by way of the arrow labelled $\phi'$ in Fig. 12, and which maps the phase axis $\phi$ to the mapped phase axis $\phi'$. The equation in the central portion of Fig. 12 may be used to map the times of signatures in the time domain, t, to the phase domain, $\phi$. The proximal time $T_0$, and the distal time $T_1$ can be mapped to corresponding angular phases within the cardiac cycle, i.e. in the range from 0 to $2\pi$ radians, by synchronising the measurement of these times to the time measurement in the measured cardiac cycle data $C_m(t)$. In the illustrated example, the proximal time $T_0$, and the distal time $T_1$ both occur in the diastole portion of the cardiac cycle.

An example of an angular phase-dependent reference fluid velocity curve $V_{ref}(\varphi)$ that is received in the operation of: receiving reference fluid velocity data comprising an angular phase-dependent reference fluid velocity curve $V_{ref}(\varphi)$ representing a fluid velocity in the vessel over a reference cardiac cycle, is illustrated in the lower left-hand portion of Fig. 12. The reference fluid velocity curve $V_{ref}(\varphi)$ is a model of the blood velocity, and may be obtained from literature, or it may be determined from clinical data. The reference fluid velocity curve $V_{ref}(\varphi)$ may represent a vessel under specified conditions, wherein reference fluid velocity curve $V_{ref}(\varphi)$ is selected to correspond to the vessel 110. For instance, the fluid velocity curve $V_{ref}(\varphi)$ may be specified for a vessel, and for each of the basal state and the hyperemic state, and for a vessel patency. This accounts for factors such as the right coronary artery generally having a markedly less pronounced diastole flow component than the left coronary artery. The vessel patency may be defined by a Fractional Flow Reserve value.

The operation of identifying in the reference fluid velocity curve $V_{ref}(\varphi)$, an angular phase of a reference, signature corresponding to each of the one or more cardiac states SS, SD in the measured cardiac cycle data $C_m(t)$; is also illustrated in the lower left-hand portion of Fig. 12. In the illustrated example, the time of a signature corresponding to the start of the systole phase, SS, is identified in the reference fluid velocity curve $V_{ref}(\varphi)$ by means of a circle symbol. Likewise, the time of a signature corresponding to the start of the diastole phase, SD, is identified in the reference fluid velocity curve $V_{ref}(\varphi)$ by means of a square symbol. The fact that these signatures in the reference fluid velocity curve $V_{ref}(\varphi)$ represent these cardiac states, may be determined from clinical studies.

The operation of transforming a phase-axis of the reference fluid velocity curve $V_{ref}(\varphi)$, is illustrated in the lower central portion of Fig. 12. The result, illustrated in the lower central portion of Fig. 12, is that the reference fluid velocity curve $V_{ref}(\varphi)$ from the lower left-hand portion of Fig. 12, is mapped onto the phase axis illustrated in the central portion of Fig. 12. This provides the transformed reference fluid velocity curve $V'_{ref}(\varphi)$. This operation may include performing a linear mapping of the phase axis of the reference fluid velocity curve $V_{ref}(\varphi)$.

Alternatively, this operation may include performing separate piecewise linear mapping of the phase axis of the reference fluid velocity curve $V_{ref}(\varphi)$ for each phase interval between the signatures of consecutive selected cardiac states.

The operation of transforming an amplitude axis of the transformed reference fluid velocity curve $V'_{ref}(\varphi)$ to provide an amplitude-transformed reference fluid velocity curve $V''_{ref}(\varphi)$, is illustrated in the lower right-hand portion of Fig. 12. This operation may include linearly scaling the amplitude axis of the transformed reference fluid velocity curve $V'_{ref}(\varphi)$. This operation may be performed iteratively until the average velocity $V_{Pref}$ of the amplitude-transformed reference fluid velocity curve $V''_{ref}(\varphi)$ computed over an angular phase interval starting at an angular phase corresponding to the proximal time $T_0$ and ending at an angular phase corresponding to the distal time $T_1$, corresponds to the average transit velocity $V_T$.

A corrected transit time $T'_T$ is then provided by multiplying the transit time $T_T$ by a ratio of the average transit velocity $V_T$ to the average velocity $V_{refFull}$ of the amplitude-transformed reference fluid velocity curve $V''_{ref}(\varphi)$ over a full cardiac cycle.

The result of these operations is to provide a corrected transit time $T'_T$ that is independent of the portion of the cardiac cycle during which the transit time $T_T$ is measured. Thus, it obviates some of the workflow complications associated with repeatedly injecting a bolus into the vessel and averaging the transit times.

Continuing with the second aspect of the present disclosure, in one example, the measured cardiac cycle data $C_m(t)$ comprises a time of a signature of each of two or more cardiac states selected from: a start of a systole cardiac phase SS, a start of a diastole cardiac phase SD, an instant of maximum velocity DM during the diastole cardiac phase, and an instant of maximum velocity during the systole cardiac phase SM, and the one or more processors are further configured to:

- transform the time axis of the reference fluid velocity curve $V_{ref}(t)$ such that the times of the reference signatures identified in the reference fluid velocity curve $V_{ref}(t)$ correspond to the times of the corresponding signatures in the measured cardiac cycle data $C_m(t)$.

Using the signature of each of two or more cardiac states in this manner improves the correspondence between the reference fluid velocity curve $V_{ret}(t)$ and the measured cardiac cycle data $C_m(t)$.

In another example, the one or more processors are configured to transform the time axis of the reference fluid velocity curve $V_{ref}(t)$ by performing a separate piecewise linear mapping of the time axis of the reference fluid velocity curve $V_{ref}(t)$ for each time interval between the signatures of consecutive selected cardiac states.

Using separate piecewise linear mappings in this manner also improves the correspondence between the reference fluid velocity curve $V_{ref}(t)$ and the measured cardiac cycle data $C_m(t)$.

The fluid velocity in vessels may depend upon various factors, including the type of vessel, e.g. LAD, LCA, the subject gender, and so forth. In another example, the one or more processors are further configured to identify the reference fluid velocity data by selecting the time-dependent reference fluid velocity curve $V_{ref}(t)$ from a database of time-dependent reference fluid velocity curves for the vessel 110.

Selecting the time-dependent reference fluid velocity curve $V_{ref}(t)$ from a database permits the use of a velocity curve $V_{ref}(t)$ that is suited to the vessel 110.

In one example, the vessel 110 forms part of a patient, and the operation of selecting the time-dependent reference fluid velocity curve $V_{ref}(t)$ from a database of time-dependent reference fluid velocity curves for the vessel 110, is based on at least one of: a type of the vessel, a dimension of the vessel, a body mass index of the patient, a gender of the patient, an age of the patient, a type of vessel dominance in the patient, an hemodynamic state of the patient, a calcification index of the patient, a plaque burden index of the patient, a vessel stiffness parameter of the patient, a cardiac function parameter of the patient.

In so doing, a velocity curve $V_{ref}(t)$ that is well-suited to the vessel 110 may be used by the system. This provides an improved corrected transit-time.

As mentioned above, the measured cardiac cycle data $C_m(t)$ may be provided by various sources. In one example, the measured cardiac cycle data $C_m(t)$ comprises at least one of:

- X-ray angiographic image data comprising a temporal sequence of images 130 representing the vessel 110;
- sensor data representing a pressure, or a velocity, or a temperature, of a fluid in the vessel 110; and
- electrocardiogram data generated by one or more sensors in communication with the heart; and
- wherein the one or more processors are configured to determine the measured cardiac period $T_{Cm}$, and the time of the signature of each of the one or more cardiac states SS, SD within the cardiac cycle, by analysing the X-ray angiographic image data, the sensor data, and the electrocardiogram data, respectively.

In some examples, the one or more processors are further configured to compute a microcirculatory resistance value, for the vessel 110, the microcirculatory resistance value being based on the transit time $T_T$, and wherein the microcirculatory resistance value is computed using the corrected transit time $T'_T$.

In one example, the microcirculatory resistance value that is computed is an index of microcirculatory resistance, IMR, value. By way of some examples, this may be calculated using Equation 1 or Equation 2. Other equations may also be used to calculate an IMR value. In one example, the one or more processors are further configured to:

- receive intraluminal device data, the intraluminal device data comprising proximal sensor data generated by a proximal sensor disposed at the proximal position $Pos_a$ in the vessel 110, and distal sensor data generated by a distal sensor disposed at the distal position $Pos_d$ in the vessel;
- and wherein the one or more processors are further configured to determine the transit time $T_T$ based on a time difference between a signal generated by the bolus at the proximal sensor and a signal generated by the bolus at the distal sensor.

By way of some examples, the proximal sensor, and the distal sensor, comprise at least one of: a pressure sensor, a fluid velocity sensor, a temperature sensor, and an ultrasound sensor. The ultrasound sensor may for example be a doppler ultrasound flow sensor.

In another example, the one or more processors are further configured to:

- receive X-ray image data, the X-ray image data comprising one or more images representing the proximal position $Pos_a$ of the proximal sensor and the distal position $Pos_d$ of the distal sensor; and
- analyse the one or more images to determine an estimate of the transit length $d_T$ based on a detection of the proximal position $Pos_a$ of the proximal sensor and the distal position $Pos_d$ of the distal sensor.

The X-ray image data may be received from an X-ray imaging system such as the X-ray imaging system 220 illustrated in Fig. 2. The proximal and distal positions may be detected in the images using a feature detector to respectively identify the proximal and distal positions. Alternatively, an artificial intelligence algorithm may be trained to detect a shape of the relevant sensor. The transit length $d_T$ may then be calculated using any of the X-ray imaging system calibration techniques described above to provide a scaling between angiographic image pixels and a real-world dimension, such as millimetres or centimetres per pixel in the patient.

In one example, the injected bolus comprises an injected contrast agent bolus 140, and wherein the one or more processors are further configured to:

- receive X-ray angiographic image data comprising a temporal sequence of images 130 representing a flow of the injected bolus 140 through the vessel 110; and
- analyse the temporal sequence of images 130 to determine the transit time $T_T$ and/or an estimate of the transit length $d_T$.

Suitable techniques for determining the transit time $T_T$ and the transit length $d_T$ are described above with reference to Fig. 5 - Fig. 9.

In one example, the one or more processors are further configured to identify the proximal position $Pos_a$ and the distal position $Pos_d$ in the vessel 110 in the X-ray angiographic image data; and

- determine the transit time $T_T$ based on the time taken for the injected contrast agent bolus 140 to travel between the identified proximal position $Pos_a$ in the vessel, and the identified distal position $Pos_d$ in the vessel, in the X-ray angiographic image data; and/or
- determine the estimate of the transit length $d_T$ based on a length of the vessel 110 between the proximal position $Pos_a$ and the distal position $Pos_d$, in the X-ray angiographic image data.

Suitable techniques for determining the transit time $T_T$ and the transit length $d_T$ are described above with reference to Fig. 5 - Fig. 9.

In one example, the proximal position $Pos_a$ corresponds to a position of a detected front of the injected contrast agent bolus 140 in the vessel 110 in an earlier X-ray image in the temporal sequence 130, and the distal position $Pos_d$ corresponds to a position of the detected front in a later X-ray image in the temporal sequence 130; and

- the transit time $T_T$ is determined by computing a time difference between the earlier X-ray image and the later X-ray image; and
- the estimate of the transit length $d_T$ is determined by:
- mapping the proximal position $Pos_a$ from the earlier image to the later image to provide a mapped proximal position $Pos'_a$ in the later image, or by mapping the distal position $Pos_d$ from the later image to the earlier image to provide a mapped distal position $Pos'_d$ in the earlier image; and
- calculating a length of the vessel 110 between the mapped proximal position $Pos'_a$ and the distal position $Pos_d$ in the later image; or between the proximal position $Pos_a$ and the mapped distal position $Pos'_d$ in the earlier image, respectively.

A suitable technique for this is described above with reference to Fig. 7.

In one example, the one or more processors are further configured to receive the transit length $d_T$ and/or the transit time $T_T$ from a computer readable storage medium, or from an intraluminal sensing device, or from a user input device. In this example, the transit time $T_T$ that is received is the transit time that is to be corrected by the system. The intraluminal sensing device may for example be a flow or pressure/ flow wire, or a thermodilution wire or another type of intraluminal device.

In another example, a method of correcting a transit time $T_T$ of a vessel 110, is provided. The transit time $T_T$ represents a time taken for an injected bolus to travel along a transit length $d_T$ of the vessel 110 between a proximal position $Pos_a$ in the vessel at a proximal time $T_0$, and a distal position $Pos_d$ in the vessel at a distal time $T_1$, and at an average transit velocity $V_T$ defined as a ratio of the transit length $d_T$ to the transit time $T_T$. The method comprises:

- receiving S210 measured cardiac cycle data $C_m(t)$ for a heart fluidically coupled to the vessel 110, the measured cardiac cycle data $C_m(t)$ comprising a measured cardiac period $T_{Cm}$, and a time $T_a$, $T_b$ of a signature of each of one or more cardiac states SS, SD within the cardiac cycle;
- mapping S220 the time of the one or more signatures, and the proximal time $T_0$, and the distal time $T_1$, to corresponding times within the measured cardiac period $T_{Cm}$;
- receiving S230 reference fluid velocity data comprising a time-dependent reference fluid velocity curve $V_{ref}(t)$ representing a fluid velocity in the vessel 110 over a reference cardiac cycle having a reference cardiac period $T_{Cref}$;
- identifying S240 in the reference fluid velocity curve $V_{ref}(t)$, a time of a reference signature corresponding to each of the one or more cardiac states SS, SD in the measured cardiac cycle data $C_m(t)$;
- transforming S250 a time axis of the reference fluid velocity curve $V_{ref}(t)$ such that the reference cardiac period $T_{Cref}$ matches the measured cardiac period $T_{Cm}$, and such that the time of the reference signature of each of the one or more cardiac states SS, SD identified in the reference fluid velocity curve $V_{ref}(t)$ corresponds to the time of the signature of each of the one or more corresponding cardiac states SS, SD in the measured cardiac cycle data $C_m(t)$;
- transforming S260 an amplitude axis of the transformed reference fluid velocity curve $V'_{ref}(t)$ to provide an amplitude-transformed reference fluid velocity curve $V''_{ref}(t)$ such that an average velocity $V_{Pref}$ of the amplitude-transformed reference fluid velocity curve $V''_{ref}(t)$ computed over a time interval between the proximal time $T_0$ and the distal time $T_1$, corresponds to the average transit velocity $V_T$; and
- providing S270 a corrected transit time $T'_T$ by multiplying the transit time $T_T$ by a ratio of the average transit velocity $V_T$ to the average velocity $V_{refFull}$ of the amplitude-transformed reference fluid velocity curve $V''_{ref}(t)$ over a full cardiac cycle.

As previously mentioned, the use of the length scale factor described above, is not limited to computing a normalised microcirculatory resistance value for a vessel. The length scale factor may also be used to compute a length of a portion of a vessel in the medical field in general. Thus, in accordance with a third aspect of the present disclosure, a system for computing a length of a portion of a vessel 110 from X-ray projection image data generated by an X-ray imaging system, is provided. Various examples, in accordance with this third aspect of the present disclosure, are enumerated as Example 1B - Example 12B below.

Example 1B. A system for computing a length of a portion of a vessel 110 from X-ray projection image data generated by an X-ray imaging system, the system comprising one or more processors configured to:

- receive S310 X-ray image data representing an X-ray projection image comprising the portion of the vessel 110;
- receive S320 X-ray imaging system geometric data representing an orientation of the X-ray imaging system respective the portion of the vessel 110;
- determine S330 a length scale factor for positions along a length of the portion of the vessel 110 in the X-ray projection image, based on a matching between the portion of the vessel in the X-ray projection image, and a reference vessel in a reference projection image generated by projecting a 3D model of a reference vessel representing the vessel 110 using the X-ray imaging system geometric data; and
- compute S340 the length of the portion of the vessel 110 by scaling the vessel 110 represented in the X-ray projection image, with the length scale factor determined at the corresponding positions along the length of the vessel 110.

Example 2B. The system according to Example 1B, wherein the one or more processors are further configured to identify the 3D model of the reference vessel 110; and wherein the one or more processors are configured to identify the 3D model by selecting a closest matching 3D model from a database of reference vessels, or by selecting a plurality of models from the database of reference vessels and combining the plurality of models to provide the 3D model.

Example 3B. The system according to Example 2B, wherein the one or more processors are configured to select the closest matching 3D model, or to select the plurality of models, based on one or more of: a length of the portion of the vessel 110, the X-ray imaging system geometric data, a cardiac phase of the vessel 110, a vessel dominance, a gender of a subject corresponding to the vessel 110, an age of a subject corresponding to the vessel 110.

Example 4B. The system according to Example 2B or Example 3B, wherein the one or more processors are further configured to:

- segment the portion of the vessel 110 in the X-ray projection image; and
- identify a centerline of the segmented portion of the vessel 110; and
- wherein the matching between the portion of the vessel 110 in the X-ray projection image, and the reference vessel in the reference projection image; is performed by matching the centerline of the segmented portion of the vessel 110 with a centerline of the reference vessel in the reference projection image.

Example 5B. The system according to any one of Example 1B - Example 4B, wherein the length of the portion of the vessel 110 is computed along a centerline of the portion of the vessel 110.

Example 6B. The system according to Example 3B or Example 4B, wherein the reference vessels in the database form part one or more coronary trees.

Example 7B. The system according to Example 6B, wherein the 3D model of the reference vessel forms part of a coronary tree;

- wherein the length scale factor at the corresponding positions along the length of the vessel 110 is averaged to provide an average length scale factor for a portion of the coronary tree; and
- wherein the computing the length of the portion of the vessel 110 by scaling the vessel represented in the X-ray projection image, with the length scale factor, comprises applying the average length scale factor to each position along the length of the vessel 110.

Example 8B. The system according to any one of Example 1B - Example 7B, wherein the received X-ray image data comprises an anatomical feature;

- wherein the 3D model of the reference vessel comprises a corresponding anatomical feature;
- wherein the projecting a 3D model of a reference vessel representing the vessel 110, comprises projecting the corresponding anatomical feature from the corresponding 3D model; and
- wherein the one or more processors are configured to determine the length scale factor for positions along a length of the vessel 110 in the X-ray projection image, based further on a scaling of a dimension of the anatomical feature in the received X-ray image data, to a dimension of the corresponding projected anatomical feature from the 3D model.

Example 9B. The system according to any one of Example 1B - Example 8B, wherein the received X-ray image data represents one and only one X-ray projection image.

Example 10B. The system according to any one of Example 1B - Example 9B, wherein the received X-ray imaging system geometric data comprises:

- a rotational angle $\alpha$ of a central ray of the X-ray imaging system 220 around a longitudinal axis of a subject 230; and/or
- a tilt angle $\beta$ of a central ray of the X-ray imaging system 220 with respect to a cranial-caudal axis of the subject 230; and/or
- a pixel size of the X-ray detector 220b of the X-ray imaging system 220; and/or
- a length or a width of a detector 220b of the X-ray imaging system 220, and a separation between an X-ray source 220a and the X-ray detector 220b of the X-ray imaging system 220, and a separation between the X-ray source 220a and the vessel 110.

Example 11B. The system according to any one of Example 1B - Example 10B, wherein the one or more processors are further configured to compute a microcirculatory resistance value for the vessel 110, the microcirculatory resistance value being based on a transit time $T_T$ taken for an injected bolus to pass between a proximal position $Pos_a$ in the vessel, and a distal position $Pos_d$ in the vessel, and wherein the one or more processors are further configured to compute the microcirculatory resistance value for the vessel 110 using an adjusted transit time $T''_T$, the adjusted transit time $T''_T$ being computed by applying to the transit time $T_T$ an adjustment factor determined by the ratio of the length of the portion of the vessel 110 computed by scaling the vessel with the length scale factor, to the length of the portion of the vessel without scaling the vessel with the length scale factor.

Example 12B. The system according to Example 11B, wherein the microcirculatory resistance value is an index of microcirculatory resistance, IMR, value.

Thus, in accordance with the third aspect of the present disclosure, a system for computing a length of a portion of a vessel 110 from X-ray projection image data generated by an X-ray imaging system, is provided. The system comprises one or more processors configured to:

- receive S310 X-ray image data representing an X-ray projection image comprising the portion of the vessel 110;
- receive S320 X-ray imaging system geometric data representing an orientation of the X-ray imaging system respective the portion of the vessel 110;
- determine S330 a length scale factor for positions along a length of the portion of the vessel 110 in the X-ray projection image, based on a matching between the portion of the vessel in the X-ray projection image, and a reference vessel in a reference projection image generated by projecting a 3D model of a reference vessel representing the vessel 110 using the X-ray imaging system geometric data; and
- compute S340 the length of the portion of the vessel 110 by scaling the vessel 110 represented in the X-ray projection image, with the length scale factor determined at the corresponding positions along the length of the vessel 110.

In so doing, an accurate length of the portion of the vessel may be computed. The computed length is accurate since it takes account of foreshortening. These operations correspond to the operations described above with reference to Fig. 13 - Fig. 16, and Fig. 2. These operations are also illustrated in Fig. 18, which is a flowchart illustrating an example of a method of computing a length of a portion of a vessel from X-ray projection image data generated by an X-ray imaging system, in accordance with some aspects of the present disclosure. Operations performed in accordance with the system for computing a length of a portion of a vessel 110, may also be performed by the method illustrated in Fig. 18, and vice versa.

With reference to Fig. 18, in the operation S310 X-ray image data representing an X-ray projection image comprising the portion of the vessel 110 is received. The X-ray image data may be received from the X-ray imaging system 220 illustrated in Fig. 2. The X-ray image data may be received via any form of data communication, including wired, optical, and wireless communication. By way of some examples, when wired or optical communication is used, the communication may take place via signals transmitted on an electrical or optical cable, and when wireless communication is used, the communication may for example be via RF or optical signals.

In the operation S320, X-ray imaging system geometric data representing an orientation of the X-ray imaging system respective the portion of the vessel 110, is received. By way of some examples, the received X-ray imaging system geometric data may include one or more of:

- a rotational angle $\alpha$ of a central ray of the X-ray imaging system around a longitudinal axis of a subject; and/or
- a tilt angle $\beta$ of the central ray of the X-ray imaging system with respect to a cranial-caudal axis of the subject; and/or
- a pixel size of the X-ray detector 220b of the X-ray imaging system 220; and/or
- a length or a width of a detector 220b of the X-ray imaging system, and a separation between an X-ray source 220a and the X-ray detector 220b of the X-ray imaging system 220, and a separation between the X-ray source 220a and the vessel 110.

Examples of a rotational angle $\alpha$, and a tilt angle $\beta$, are described above with reference to Fig 14 and Fig. 16.

In the operation S330, a length scale factor for positions along a length of the portion of the vessel 110 in the X-ray projection image, is determined based on a matching between the portion of the vessel in the X-ray projection image, and a reference vessel in a reference projection image generated by projecting a 3D model of a reference vessel representing the vessel 110 using the X-ray imaging system geometric data. These operations are described above with reference to Fig. 13 and Fig. 14.

In the operation S340, the length of the portion of the vessel 110 is computed by scaling the vessel 110 represented in the X-ray projection image, with the length scale factor determined at the corresponding positions along the length of the vessel 110. These operations are also described above with reference to Fig. 13 and Fig. 14. In so doing, an accurate length of the portion of the vessel 110 may be computed.

In one example, the one or more processors are further configured to identify the 3D model of the reference vessel 110; and the one or more processors are configured to identify the 3D model by selecting a closest matching 3D model from a database of reference vessels, or by selecting a plurality of models from the database of reference vessels and combining the plurality of models to provide the 3D model.

Various factors may be taken into account in identifying the 3D model. By way of some examples, the one or more processors may select the closest matching 3D model, or to select the plurality of models, based on one or more of: a length of the portion of the vessel 110, the X-ray imaging system geometric data, a cardiac phase of the vessel 110, a vessel dominance, a gender of a subject corresponding to the vessel 110, an age of a subject corresponding to the vessel 110.

For example, if a 3D model and the current vessel 110 correspond to the same cardiac phase, once registered, for instance from the registration of a proximal location, vessel matching can be performed based on a computed distance between the curves represented by the model and the current vessel 110. The distance may be computed using the Euclidian distance between sampled points, for instance. However, if the 3D model and the current vessel 110 correspond to different portions of the cardiac cycle, then the vessel projections will have poorer correspondence. In this case, matching may be achieved by comparing angles and/or curvatures of the vessel. Similarly, the positions of bifurcations may be used to compare the 3D model and the current vessel 110 based on the locations of one or more bifurcation.

The database of reference vessels may also include 3D models of vessels relating to different cardiac phases. In this case, the processor(s) would select only the 3D models having the same cardiac phase as the vessel 110.

In one example, the one or more processors are further configured to:

- segment the portion of the vessel 110 in the X-ray projection image; and
- identify a centerline of the segmented portion of the vessel 110; and
- wherein the matching between the portion of the vessel 110 in the X-ray projection image, and the reference vessel in the reference projection image; is performed by matching the centerline of the segmented portion of the vessel 110 with a centerline of the reference vessel in the reference projection image.

Using the centerlines of segmented vessels in this manner has been found to provide an accurate technique for determining the length scale factor.

In one example, the length of the portion of the vessel 110 is computed along a centerline of the portion of the vessel 110. Computing the length of the portion of the vessel using the centerlines has been found to provide an accurate length.

In one example, the reference vessels in the database form part one or more coronary trees. In this example, the 3D model of the reference vessel may form part of a coronary tree. In this example, the length scale factor at the corresponding positions along the length of the vessel 110 is averaged to provide an average length scale factor for a portion of the coronary tree; and the computing the length of the portion of the vessel 110 by scaling the vessel represented in the X-ray projection image, with the length scale factor, comprises applying the average length scale factor to each position along the length of the vessel 110.

In one example, the received X-ray image data comprises an anatomical feature. The anatomical feature may represent the heart, a bone, and so forth. In this example, the 3D model of the reference vessel comprises a corresponding anatomical feature, and the operation of projecting a 3D model of a reference vessel representing the vessel 110, comprises projecting the corresponding anatomical feature from the corresponding 3D model. Moreover, in this example, the one or more processors are configured to determine the length scale factor for positions along a length of the vessel 110 in the X-ray projection image, based further on a scaling of a dimension of the anatomical feature in the received X-ray image data, to a dimension of the corresponding projected anatomical feature from the 3D model.

By way of an example, the feature may be a shadow of the heart, and which is often visible in X-ray images. Since the dimension of the anatomical feature is known, it may be used to calibrate the length scale factor.

In one example, the received X-ray image data represents one and only one X-ray projection image.

In one example, the one or more processors are further configured to compute a microcirculatory resistance value for the vessel 110, the microcirculatory resistance value being based on a transit time $T_T$ taken for an injected bolus to pass between a proximal position $Pos_a$ in the vessel, and a distal position $Posd$ in the vessel, and wherein the one or more processors are further configured to compute the microcirculatory resistance value for the vessel 110 using an adjusted transit time $T''_T$, the adjusted transit time $T''_T$ being computed by applying to the transit time $T_T$ an adjustment factor determined by the ratio of the length of the portion of the vessel 110 computed by scaling the vessel with the length scale factor, to the length of the portion of the vessel without scaling the vessel with the length scale factor.

In one example, the microcirculatory resistance value is an index of microcirculatory resistance, IMR, value. The IMR value may be computed using Equation 1 or Equation 2 above. Other equations may also be used to calculate an IMR value.

In another example, a method of computing a length of a portion of a vessel 110 from X-ray projection image data generated by an X-ray imaging system, is provided. The method comprises:

- receiving S310 X-ray image data representing an X-ray projection image comprising the portion of the vessel 110;
- receiving S320 X-ray imaging system geometric data representing an orientation of the X-ray imaging system respective the portion of the vessel 110;
- determining S330 a length scale factor for positions along a length of the portion of the vessel 110 in the X-ray projection image, based on a matching between the portion of the vessel in the X-ray projection image, and a reference vessel in a reference projection image generated by projecting a 3D model of a reference vessel representing the vessel 110 using the X-ray imaging system geometric data; and
- computing S340 the length of the portion of the vessel 110 by scaling the vessel 110 represented in the X-ray projection image, with the length scale factor determined at the corresponding positions along the length of the vessel 110.

In accordance with a fourth aspect of the present disclosure, a system for providing value of a transit velocity of an injected contrast agent bolus 140 in a vessel, is provided. In this fourth aspect, the vessel forms part of a vessel tree. Various examples, in accordance with this third aspect of the present disclosure, are enumerated as Example 1C - Example 3C below.

Example 1C. A system for providing value of a transit velocity of an injected contrast agent bolus 140 in a vessel, the system comprising one or more processors configured to:

- receive X-ray angiographic image data comprising a temporal sequence of images 130 representing a flow of the injected contrast agent bolus 140 through a vessel tree comprising the vessel;
- generate a time-intensity curve for the vessel tree from the temporal sequence of images 130;
- calculate a transit time $T_T$ from the time-intensity curve, the transit time being defined by a difference between a first point in time $T_{0a}$ at which the bolus 140 enters a portion of the vessel tree, and a second point in time $T_{1a}$ at which the bolus 140 saturates the vessel tree, or the vessel, in the corresponding images in the temporal sequence 130;
- identify an earlier X-ray image corresponding to the first point in time $T_{0a}$;
- identify a later X-ray image corresponding to the second point in time $T_{1a}$;
- determine an estimate of the transit length $d_T$ of the vessel based on an analysis of the earlier X-ray image and the later X-ray image, the transit length $d_T$ corresponding to a length of the vessel traversed by the bolus 140 between the first point in time $T_{0a}$ and the second point in time $T_{1a}$; and
- calculate the value of the transit velocity of the injected contrast agent bolus 140 in the vessel based on a ratio of the estimated transit length $d_T$ to the calculated transit time $T_T$.

Example 2C. The system according to Example 1C, wherein the one or more processors are configured to determine an estimate of the transit length $d_T$ of the vessel by:

- detecting a first position A of a front of the injected contrast agent bolus 140 in the vessel in the earlier X-ray image, and a second position B of the front in the later X-ray image;
- mapping the first position A from the earlier image to the later image to provide a mapped first position A' in the later image, or mapping the second position B from the later image to the earlier image to provide a mapped second position B' in the earlier image; and
- computing the estimate of the transit length $d_T$ as the length between the mapped first position A' and the second position B in the later image, or between the first position A and the mapped second position B' in the earlier image, respectively.

Example 3C. The system according to Example 1C or Example 2C, wherein the second position B corresponds to the extremity of the longest path in the vessel tree, starting from the proximal position in the second X-ray image.

The operations performed in Example 3A - Example 3C are described above with reference to Fig. 8 - Fig. 10.

The above examples are to be understood as illustrative of the present disclosure, and not restrictive. Further examples are also contemplated. For instance, the examples described in relation to a system, may also be provided by a computer program product, or by a computer-readable storage medium, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

**Claims**

1. A system for providing value of a transit velocity of an injected contrast agent bolus in a lumen, the system comprising one or more processors configured to:

   receive X-ray angiographic image data comprising a temporal sequence of images representing a flow of the injected contrast agent bolus through a vessel tree comprising the lumen;
   generate a time-intensity curve for the vessel tree from the temporal sequence of images;
   calculate a transit time ($T_T$) from the time-intensity curve, the transit time being defined by a difference between a first point in time (T1) at which the bolus enters a portion of the vessel tree, and a second point in time (T2) at which the bolus saturates the vessel tree, or the lumen, in the corresponding images in the temporal sequence;
   identify an earlier X-ray image corresponding to the first point in time (T1);
   identify a later X-ray image corresponding to the second point in time (T2);
   determine an estimate of the transit length ($d_T$) of the lumen based on an analysis of the earlier X-ray image and the later X-ray image, the transit length ($d_T$) corresponding to a length of the lumen traversed by the bolus between the first point in time (T1) and the second point in time (T2); and
   calculate the value of the transit velocity of the injected contrast agent bolus in the lumen based on a ratio of the estimated transit length ($d_T$) to the calculated transit time ($T_T$).

2. The system according to claim 1, wherein the one or more processors are configured to determine an estimate of the transit length ($d_T$) of the lumen by:

   detecting a first position of a front of the injected contrast agent bolus in the lumen in the earlier X-ray image, and a second position of the front in the later X-ray image;
   mapping the first position from the earlier image to the later image, or mapping the second position from the later image to the earlier image; and
   computing the estimate of the transit length ($d_T$) as the length between the first position and the second position in the later image, or in the earlier image, respectively.

3. The system according to claim 1, wherein the second position corresponds to the extremity of the longest path in the vessel tree, starting from the proximal position in the second X-ray image.

4. A system for correcting a transit time ($T_T$) of a vessel (110), the transit time ($T_T$) representing a time taken for an injected bolus to travel along a transit length ($d_T$) of the vessel (110) between a proximal position ($Pos_a$) in the vessel at a proximal time ($T_0$), and a distal position ($Pos_d$) in the vessel at a distal time ($T_1$), and at an average transit velocity ($V_T$) defined as a ratio of the transit length ($d_T$) to the transit time ($T_T$), the system comprising one or more processors configured to:

> receive (S210) measured cardiac cycle data ($C_m(t)$) for a heart fluidically coupled to the vessel (110), the measured cardiac cycle data ($C_m(t)$) comprising a measured cardiac period ($T_{Cm}$), and a time ($T_a$, $T_b$) of a signature of each of one or more cardiac states (SS, SD) within the cardiac cycle;
> map (S220) the time of the one or more signatures, and the proximal time ($T_0$), and the distal time ($T_1$), to corresponding times within the measured cardiac period ($T_{Cm}$);
> receive (S230) reference fluid velocity data comprising a time-dependent reference fluid velocity curve ($V_{ref}(t)$) representing a fluid velocity in the vessel (110) over a reference cardiac cycle having a reference cardiac period ($T_{Cref}$);
> identify (S240) in the reference fluid velocity curve ($Vret(t)$), a time of a reference signature corresponding to each of the one or more cardiac states (SS, SD) in the measured cardiac cycle data ($C_m(t)$);
> transform (S250) a time axis of the reference fluid velocity curve ($V_{ref}(t)$) such that the reference cardiac period ($T_{Cref}$) matches the measured cardiac period ($T_{Cm}$), and such that the time of the reference signature of each of the one or more cardiac states (SS, SD) identified in the reference fluid velocity curve ($V_{ref}(t)$) corresponds to the time of the signature of each of the one or more corresponding cardiac states (SS, SD) in the measured cardiac cycle data ($C_m(t)$);
> transform (S260) an amplitude axis of the transformed reference fluid velocity curve ($V'_{ref}(t)$) to provide an amplitude-transformed reference fluid velocity curve ($V''_{ref}(t)$) such that an average velocity ($V_{Pref}$) of the amplitude-transformed reference fluid velocity curve ($V''_{ref}(t)$) computed over a time interval between the proximal time ($T_0$) and the distal time ($T_1$), corresponds to the average transit velocity ($V_T$); and
> provide (S270) a corrected transit time ($T'_T$) by multiplying the transit time ($T_T$) by a ratio of the average transit velocity ($V_T$) to the average velocity ($V_{refFull}$) of the amplitude-transformed reference fluid velocity curve ($V''_{ref}(t)$) over a full cardiac cycle.

5. The system according to claim 4, wherein the measured cardiac cycle data ($C_m(t)$) comprises a time of a signature of each of two or more cardiac states selected from: a start of a systole cardiac phase (SS), a start of a diastole cardiac phase (SD), an instant of maximum velocity (DM) during the diastole cardiac phase, and an instant of maximum velocity during the systole cardiac phase (SM), and wherein the one or more processors are further configured to:

transform the time axis of the reference fluid velocity curve ($V_{ref}(t)$) such that the times of the reference signatures identified in the reference fluid velocity curve ($V_{ref}(t)$) correspond to the times of the corresponding signatures in the measured cardiac cycle data ($C_m(t)$).

6. The system according to claim 5, wherein the one or more processors are configured to transform the time axis of the reference fluid velocity curve ($V_{ref}(t)$) by performing a separate piecewise linear mapping of the time axis of the reference fluid velocity curve ($Vret(t)$) for each time interval between the signatures of consecutive selected cardiac states.

7. The system according to any one of claims 4 - 6, wherein the one or more processors are further configured to identify the reference fluid velocity data by selecting the time-dependent reference fluid velocity curve ($V_{ref}(t)$) from a database of time-dependent reference fluid velocity curves for the vessel.

8. The system according to claim 7, wherein the vessel forms part of a patient, and wherein the selecting the time-dependent reference fluid velocity curve ($V_{ref}(t)$) from a database of time-dependent reference fluid velocity curves for the vessel, is based on at least one of: a type of the vessel, a dimension of the vessel, a body mass index of the patient, a gender of the patient, an age of the patient, a type of vessel dominance in the patient, an hemodynamic state of the patient, a calcification index of the patient, a plaque burden index of the patient, a vessel stiffness parameter of the patient, a cardiac function parameter of the patient.

9. The system according to any one of claims 4 - 8, wherein the measured cardiac cycle data ($C_m(t)$) comprises at least one of:

> X-ray angiographic image data comprising a temporal sequence of images representing the vessel;

sensor data representing a pressure, or a velocity, or a temperature, of a fluid in the vessel; and electrocardiogram data generated by one or more sensors in communication with the heart; and

wherein the one or more processors are configured to determine the measured cardiac period ($T_{Cm}$), and the time of the signature of each of the one or more cardiac states (SS, SD) within the cardiac cycle, by analysing the X-ray angiographic image data, the sensor data, and the electrocardiogram data, respectively.

10. The system according to any one of claims 4 - 9, wherein the one or more processors are further configured to compute a microcirculatory resistance value, for the vessel, the microcirculatory resistance value being based on the transit time ($T_T$), and wherein the microcirculatory resistance value is computed using the corrected transit time ($T'_T$).

11. The system according to claim 10, wherein the microcirculatory resistance value is an index of microcirculatory resistance, IMR, value.

12. The system according to any one of claims 4 - 11, wherein the one or more processors are further configured to:

receive intraluminal device data, the intraluminal device data comprising proximal sensor data generated by a proximal sensor disposed at the proximal position in the vessel, and distal sensor data generated by a distal sensor disposed at the distal position in the vessel;

and wherein the one or more processors are further configured to determine the transit time ($T_T$) based on a time difference between a signal generated by the bolus at the proximal sensor and a signal generated by the bolus at the distal sensor.

13. The system according to any one of claims 4 - 12, wherein the proximal sensor, and the distal sensor, comprise at least one of: a pressure sensor, a fluid velocity sensor, a temperature sensor, and an ultrasound sensor.

14. The system according to claim 12 or claim 13, wherein the one or more processors are further configured to:

receive X-ray image data, the X-ray image data comprising one or more images representing the proximal position of the proximal sensor and the distal position of the distal sensor; and

analyse the one or more images to determine an estimate of the transit length ($d_T$) based on a detection of the proximal position of the proximal sensor and the distal position of the distal sensor.

15. The system according to any one of claims 4 - 11, wherein the injected bolus comprises an injected contrast agent bolus, and wherein the one or more processors are further configured to:

receive X-ray angiographic image data comprising a temporal sequence of images representing a flow of the injected bolus through the vessel; and

analyse the temporal sequence of images to determine the transit time ($T_T$) and/or an estimate of the transit length ($d_T$).

FIG. 1

FIG. 2

S110

↓

S120

# FIG. 3

$A1_{Trigger}$

$I_1$

140

Time (t)

$T1_0$   $T1_1$

$T_{SEQ}$

130

110

# FIG. 4

FIG. 5

EP 4 162 879 A1

130

$T_0$

$T_1$

$Pos_a, T_0$

110

$Pos_d, T_1$

FIG. 6

$T_0$

$T_1$

$Pos_a, T_0$

$Pos'_a, T_1$

110

$d_T$

$Pos_d, T_1$

EP 4 162 879 A1

# FIG. 7

FIG. 8

FIG. 9

FIG. 10

FIG. 11

$$t' = TCre_f \frac{mod(t, T_{Cm})}{T_{Cm}}$$

$$V''_{ref}(t) = \alpha \, V_{ref}(t')$$

$$V'_{ref}(t) = V_{ref}(t')$$

Time-axis transform

Amplitude axis transform

EP 4 162 879 A1

FIG. 12

FIG. 13

FIG. 14

**FIG. 15**

**FIG. 16**

S210

↓

S220

↓

S230

↓

S240

↓

S250

↓

S260

↓

S270

# FIG. 17

S310

↓

S320

↓

S330

↓

S340

# FIG. 18

Europäisches
Patentamt

European
Patent Office

Office européen
des brevets

## EUROPEAN SEARCH REPORT

Application Number

EP 21 29 0060

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/148662 A1 (ALEX JULIE [IN]) 28 May 2015 (2015-05-28) | 1 | INV. A61B6/00 |
| Y | * paragraph [0041] – paragraph [0092]; figures * | 3 | |
| X | EP 3 403 582 A1 (PIE MEDICAL IMAGING BV [NL]) 21 November 2018 (2018-11-21) * paragraph [0022] – paragraph [0038] * * paragraph [0042] – paragraph [0061]; figures * | 1,2 | |
| X | US 2019/038249 A1 (ITU LUCIAN MIHAI [RO] ET AL) 7 February 2019 (2019-02-07) * paragraph [0103] – paragraph [0107]; figures * | 1 | |
| Y | US 2006/239528 A1 (CAMUS ESTELLE [DE] ET AL) 26 October 2006 (2006-10-26) * paragraph [0034] – paragraph [0058]; figures * | 3 | |

TECHNICAL FIELDS
SEARCHED     (IPC)

A61B

The present search report has been drawn up for all claims

| Place of search | Date of completion of the search | Examiner |
|---|---|---|
| Munich | 21 March 2022 | Strubel, Christine |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

.................................................................

& : member of the same patent family, corresponding
    document

EPO FORM 1503 03.82 (P04C01)

Europäisches
Patentamt

European
Patent Office

Office européen
des brevets

**Application Number**

EP 21 29 0060

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-3

☐ The present supplementary European search report has been drawn up for those parts of the European patent application which relate to the invention first mentioned in the claims (Rule 164 (1) EPC).

**Europäisches Patentamt**
**European Patent Office**
**Office européen des brevets**

# LACK OF UNITY OF INVENTION
# SHEET B

**Application Number**

**EP 21 29 0060**

The Search Division considers that the present European patent application does not comply with the requirements of unity of invention and relates to several inventions or groups of inventions, namely:

```
     1. claims: 1-3

          A system for providing value of a transit velocity of an
          injected contrast agent bolus
                         ---


     2. claims: 4-15

          A system for correcting a transit time of a vessel
                         ---
```

## ANNEX TO THE EUROPEAN SEARCH REPORT
## ON EUROPEAN PATENT APPLICATION NO.

EP 21 29 0060

This annex lists the patent family members relating to the patent documents cited in the above-mentioned European search report.
The members are as contained in the European Patent Office EDP file on
The European Patent Office is in no way liable for these particulars which are merely given for the purpose of information.

21-03-2022

| Patent document cited in search report | | Publication date | Patent family member(s) | | Publication date |
|---|---|---|---|---|---|
| US 2015148662 | A1 | 28-05-2015 | NONE | | |
| EP 3403582 | A1 | 21-11-2018 | EP | 3403582 A1 | 21-11-2018 |
| | | | JP | 2019022647 A | 14-02-2019 |
| | | | US | 2018330507 A1 | 15-11-2018 |
| US 2019038249 | A1 | 07-02-2019 | CN | 106037710 A | 26-10-2016 |
| | | | US | 9349178 B1 | 24-05-2016 |
| | | | US | 2016148372 A1 | 26-05-2016 |
| | | | US | 2017245821 A1 | 31-08-2017 |
| | | | US | 2018153495 A1 | 07-06-2018 |
| | | | US | 2019038249 A1 | 07-02-2019 |
| | | | US | 2021219935 A1 | 22-07-2021 |
| US 2006239528 | A1 | 26-10-2006 | CN | 1864646 A | 22-11-2006 |
| | | | DE | 102005018327 A1 | 26-10-2006 |
| | | | JP | 2006297102 A | 02-11-2006 |
| | | | US | 2006239528 A1 | 26-10-2006 |

EPO FORM P0459

For more details about this annex : see Official Journal of the European Patent Office, No. 12/82

## REFERENCES CITED IN THE DESCRIPTION

**Non-patent literature cited in the description**

- **KOBAYASHI, Y. ; FEARON, W. F.** Invasive coronary microcirculation assessment - Current status of Index of Microcirculatory Resistance. *Circulation Journal, Official Journal of the Japanese Circulation Society,* 2014, vol. 78 (5), 1021-1028
- **KOBAYASHI, Y. ; FEARON, W. F.** Invasive coronary microcirculation assessment - Current status of Index of Microcirculatory Resistance. *Circulation Journal, Official Journal of the Japanese Circulation Society,* 2014, vol. 785, 1021-1028

- **IYER, K. et al.** AngioNet: A Convolutional Neural Network for Vessel Segmentation in X-ray Angiography. *medRxiv 2021.01.25.21250488,* 25 January 2021, 21250488
- A reporting system on patients evaluated for coronary artery disease. **AUSTEN, W. G. et al.** Circulation. Council on Cardiovascular Surgery, American Heart Association, 1975, vol. 51, 5-40